# EUROPEAN PATENT APPLICATION

(11) **EP 4 699 601 A2**
(43) Date of publication of application: **25.02.2026**
(21) Application number: 25213249.3
(22) Date of filing: 13.09.2017
(51) Int. Cl.: A61K 9/19

(54) **NON-PROTEIN CLOSTRIDIAL TOXIN COMPOSITIONS**

(30) Priority: 13.09.2016 US 201662394009 P
(62) Divisional of application: 19190978.7
(71) Applicant: Allergan, Inc., Irvine, CA 92612 (US)
(72) Inventor: Abiad, Maurice, Anaheim, CA California, 92804 (US); Dani, Bhas, San Diego, CA California, 92130 (US); Shalaev, Evgenyi, Dana Point, CA California, 92629 (US)
(74) Representative: Hoffmann Eitle

(57) **Abstract**

Pharmaceutical compositions that stabilize a Clostridial toxin active ingredient are described. The compositions can be liquid or solid compositions, and comprise a surfactant and an antioxidant. In some embodiments, the compositions comprise a surfactant selected from a poloxamer and a polysorbate; an antioxidant selected from methionine, N-acetyl cysteine, ethylenediaminetetraacetic acid and combinations thereof; and, optionally, a tonicity agent and/or a lyoprotector selected from, for example, trehalose and sucrose.

## Description

### FIELD

The present invention relates to solid and liquid pharmaceutical compositions comprising a clostridial toxin active ingredient and one or more non-protein excipient.

### BACKGROUND

A pharmaceutical composition is a formulation which contains at least one active ingredient (such as a Clostridial toxin) as well as, for example, one or more excipients, buffers, carriers, stabilizers, preservatives and/or bulking agents, and is suitable for administration to a patient to achieve a desired diagnostic result or therapeutic effect. The pharmaceutical compositions disclosed herein have diagnostic, therapeutic and/or research utility.

For storage stability and convenience of handling, a pharmaceutical composition can be formulated as a lyophilized (i.e. freeze dried) or vacuum dried powder which can be reconstituted with a suitable fluid, such as saline or water, prior to administration to a patient. Alternately, the pharmaceutical composition can be formulated as an aqueous solution or suspension. A pharmaceutical composition can contain a proteinaceous active ingredient. Unfortunately, a protein active ingredient can be very difficult to stabilize (i.e. maintained in a state where loss of biological activity is minimized), thereby resulting in a loss of protein and/or loss of protein activity during the formulation, reconstitution (if required) and storage of the pharmaceutical composition prior to use. Stability problems can arise due to surface adsorption of a protein active ingredient, physical instability, such as, *e.g.,* denaturation or aggregation, or chemical instability, such as, *e.g.,* cross-linking, deamidation, isomerization, oxidation, formation of acidic or basic species, Maillard reaction, and fragmentation. To prevent such instability, various protein-based excipients, such as albumin and gelatin, have been used to stabilize a protein active ingredient present in a pharmaceutical composition.

Unfortunately, despite their known stabilizing effects, significant drawbacks exist to the use of protein excipients, such as albumin or gelatin, in a pharmaceutical composition. For example albumin and gelatin are expensive and increasingly difficult to obtain. Furthermore, blood products or animal derived products such as albumin and gelatin, when administered to a patient can subject the patient to a potential risk of receiving blood borne pathogens or infectious agents. Thus, it is known that the possibility exists that the presence of an animal-derived protein excipient in a pharmaceutical composition can result in inadvertent incorporation of infectious elements into the pharmaceutical composition. For example, it has been reported that use of human serum albumin may transmit prions into a pharmaceutical composition. Thus, it is desirable to find suitable non-protein excipients, such as, e.g., stabilizers, cryo-protectants and lyo-protectants, which can be used to stabilize the protein active ingredient present in a pharmaceutical composition.

The unique characteristics of Clostridial toxins further constrain and hinder the selection of suitable non-protein excipients for a pharmaceutical composition comprising a Clostridial toxin active ingredient. For example, Clostridial toxins are large proteins having an average molecular weight of approximately 150 kDa, and are further complexed with non-toxin associated proteins that increase the size to approximately 300-900-kDa. The size of a Clostridial toxin complex makes it much more fragile and labile than smaller, less complex proteins, thereby compounding the formulation and handling difficulties if Clostridial toxin stability is to be maintained. Hence, the use of non-protein excipients, such as, *e.g.,* stabilizers, cryo-protectants and lyo-protectants must be able to interact with the Clostridial toxin active ingredient in a manner which does not denature, fragment or otherwise inactivate the toxin or cause disassociation of the non-toxin associated proteins present in the toxin complex.

Another problem associated with a Clostridial toxin active ingredient, is the exceptional safety, precision, and accuracy that is necessary for at all steps of the formulation process. Thus, a non-protein excipient should not itself be toxic or difficult to handle so as to not exacerbate the already extremely stringent requirements.

Still another difficulty linked with a Clostridial toxin active ingredient, is the incredible low amounts of Clostridial toxin that is used in a pharmaceutical composition. As with enzymes generally, the biological activities of the Clostridial toxins are dependant, at least in part, upon their three dimensional conformation. Thus, a Clostridial toxin is detoxified by heat, various chemicals, surface stretching, and surface drying. Additionally, it is known that dilution of a Clostridial toxin complex obtained by the known culturing, fermentation and purification methods to the much lower concentration used in a pharmaceutical composition results in rapid inactivation of the toxin. The extremely low amount of a Clostridial toxin active ingredient that is used in a pharmaceutical composition, makes this active ingredient very susceptible to adsorption to, *e.g.,* the surfaces of laboratory glassware, vessels, to the vial in which the pharmaceutical composition is reconstituted and to the inside surface of a syringe used to inject the pharmaceutical composition. Such adsorption of a Clostridial toxin active ingredient to surfaces can lead to a loss of active ingredient and to denaturation of the remaining Clostridial toxin active ingredient, both of which reduce the total activity of the active ingredient present in the pharmaceutical composition. Hence, the use of non-protein excipients, such as, *e.g.,* stabilizers, cryo-protectants and lyo-protectants must be able to act as surface blockers to prevent the adsorption of a Clostridial toxin active ingredient to a surface.

Yet another problem connected to a Clostridial toxin active ingredient, is the pH-sensitivity associates with complex formation. For example, the 900-kDa BoNT/A complex is known to be soluble in dilute aqueous solutions at pH 3.5-6.8. However, at a pH above about 7 the non-toxic associated proteins dissociate from the 150-kDa neurotoxin, resulting in a loss of toxicity, particularly as the pH rises above pH 8.0. See Edward J. Schantz et al., pp. 44-45, Preparation and characterization of botulinum toxin type A for human treatment, in Jankovic, J., et al., Therapy with Botulinum Toxin (Marcel Dekker, Inc., 1994). As the non-toxic associated proteins are believed to preserve or help stabilize the secondary and tertiary structures upon which toxicity is depends, the dissociation of these proteins results in a more unstable Clostridial toxin active ingredient. Thus, non-protein excipients useful to formulate a pharmaceutical composition comprising a Clostridial toxin active ingredient must be able to operate within the confines of a pH level necessary to maintain the activity a Clostridial toxin active ingredient.

What is needed therefore is a Clostridial toxin pharmaceutical composition wherein a Clostridial toxin active ingredient (such as a botulinum toxin) is stabilized by a non-protein excipient. The present invention relates to solid and liquid Clostridial toxin pharmaceutical compositions with one or more non-protein excipients which functions to stabilize the Clostridial toxin active ingredient present in the solid or liquid pharmaceutical composition.

### SUMMARY

In one aspect, there is provided a pharmaceutical composition comprising a Clostridial toxin active ingredient, a tonicity agent, a surfactant and an antioxidant. In some embodiments, the pharmaceutical compositions comprisies a botulinum toxin. In some embodiments, the pharmaceutical composition comprises trehalose. In some embodiments, the pharmaceutical composition comprises sodium chloride. In some embodiments, the composition comprises a poloxamer and/or a polysorbate. In some embodiments, the composition comprises poloxamer 188 and/or polysorbate 20. In some embodiments, the antioxidant is selected from the group consisting of L-methionine, N-Acetyl-cystein (NAC), butylated hydroxytoluene (BHT), ethylene diamine tetraacetic acid sodium salt (EDTA), an EDTA analog, ethylene glycol-bis(2-aminoethylether)-N,N,N',N'-tetraacetic acid (EGTA), an EGTA analog, diethylenetriaminepentaacetic acid (DTPA), a DTPA analog, ascorbic acid, and combinations thereof. In some embodiments, the antioxidant is methionine. In some embodiments, the antioxidant is NAC. In some embodiments, the antioxidant is NAC and EDTA. In some embodiments, the composition further comprises a buffering agent. In one embodiment, the buffering agent includes histidine buffer. In some embodiments, the composition has a pH of from 5 to 7. In some embodiments, the composition is a liquid formulation. In some embodiments, the composition is a solid formulation.

In one aspect, the present disclosure provides a liquid pharmaceutical composition comprising a Clostridial toxin active ingredient, trehalose, poloxamer 188 or polysorbate 20, and L-methionine or N-acetyl-cysteine (NAC). In some embodiments, the liquid pharmaceutical composition comprises a botulinum toxin. In some embodiments, the liquid pharmaceutical composition further comprises EDTA or an EDTA analog. In some embodiments, the liquid pharmaceutical composition comprises a histidine buffer. In some embodiments, the pH of the liquid pharmaceutical composition ranges from 5 to 7. In some embodiments, the relative weight amount of L-methionine ranges from about 0.1% to about 0.3%. In some embodiments, the relative weight amount of NAC ranges from about 0.1% to about 0.5%. In some embodiments, the relative weight amount of EDTA ranges from about 0.01% to about 0.05%. In some embodiments, the relative weight amount of trehalose ranges from about 1.0 to about 10%. In some embodiments, the relative weight amount of poloxamer 188 ranges from about 2% to about 5%. In some embodiments, the relative weight amount of polysorbate 20 ranges from about 0.02% to about 0.06%.

In another aspect, the present disclosure provides a solid pharmaceutical composition comprising a botulinum toxin, trehalose, poloxamer 188 or polysorbate 20, NAC, and EDTA or an EDTA analog. In an alternative embodiment, the solid pharmaceutical composition comprises a botulinum toxin, trehalose, poloxamer 188, and NAC. In an alternative embodiment, the solid pharmaceutical composition comprises a botulinum toxin, trehalose, poloxamer 188 and L-methionine. In some embodiments, the solid pharmaceutical composition further comprises histidine buffer. In some embodiments, the relative weight amount of L-methionine ranges from about 0.1% to about 0.3%. In some embodiments, the relative weight amount of NAC ranges from about 0.01% to about 0.05%. In some embodiments, the relative weight amount of EDTA ranges from about 0.01% to about 0.05%. In some embodiments, the relative weight amount of trehalose ranges from about 1.0 to about 10%. In some embodiments, the relative weight amount of poloxamer 188 ranges from about 0.5% to about 5%. In some embodiments, the relative weight amount of polysorbate 20 ranges from about 0.02% to about 0.06%.

### DESCRIPTION

Certain compositions of the present invention provide stable liquid or solid pharmaceutical composition comprising a clostridical toxin active ingredient, a disaccharide, a surfactant and an antioxidant. In certain liquid compositions of the present invention, the disaccharide is optional.

Certain embodiments also provide methods for the treatment of various diseases, disorders, and conditions, including, for example, depression (e.g. major depressive disorder), headache (e.g. migraine, tension headache, and the like), pain, atrial fibrillation, hyperhidrosis, muscle spasticity, cervical dystonia, blepherospasm, overactive bladder (e.g. neurogenic detrusor overactivity, and idiopathic overactive bladder), bladder pain (e.g. interstitial cystitis, bladder pain syndrome), skin conditions (e.g. wrinkles, fine wrinkles, excess sebum production, acne, rosacea), irregularities, and the like using the compositions provided according to aspect of the present invention. Embodiments can include various administration techniques, including, for example, injection, such as intramusclular, intracutaneous, subcutaneous, or the like, instillation, intravenous, transdermal, and topical.

### Definitions

As used herein, the words or terms set forth below have the following definitions:
The articles "a" and "an" are used herein to refer to one or to more than one (i.e., to at least one) of the grammatical object of the article. By way of example, "an element" means one element or more than one element.

"About" or "approximately" as used herein means within an acceptable error range for the particular value as determined by one of ordinary skill in the art, which will depend in part on how the value is measured or determined, (*i.e.*, the limitations of the measurement system). For example, "about" can mean within 1 or more than 1 standard deviations, per practice in the art. Where particular values are described in the application and claims, unless otherwise stated, the term "about" means within an acceptable error range for the particular value.

"Administration", or "to administer" means the step of giving (i.e. administering) a pharmaceutical composition to a subject, or alternatively a subject receiving a pharmaceutical composition. The pharmaceutical compositions disclosed herein can be locally administered by various methods. For example, intramuscular, intradermal, subcutaneous administration, intrathecal administration, intraperitoneal administration, topical (transdermal), instillation, and implantation (for example, of a slow-release device such as polymeric implant or miniosmotic pump) can all be appropriate routes of administration.

"Alleviating" means a reduction in the occurrence of a pain, of a headache, or of any symptom or cause of a condition or disorder. Thus, alleviating includes some reduction, significant reduction, near total reduction, and total reduction.

"Animal protein free" means the absence of blood derived, blood pooled and other animal derived products or compounds. "Animal" means a mammal (such as a human), bird, reptile, fish, insect, spider or other animal species. "Animal" excludes microorganisms, such as bacteria. Thus, an animal protein free pharmaceutical composition can include a botulinum neurotoxin. For example, an "animal protein free" pharmaceutical composition means a pharmaceutical composition which is either substantially free or essentially free or entirely free of a serum derived albumin, gelatin and other animal derived proteins, such as immunoglobulins. An example of an animal protein free pharmaceutical composition is a pharmaceutical composition which comprises or which consists of a botulinum toxin (as the active ingredient) and a suitable polysaccharide as a stabilizer or excipient.

"Antioxidant" refers to any compound which protects an active ingredient from reaction with oxygen. Antioxidants can be broadly divided into three categories: (i) sacrificial antioxidants, which react with oxygen more readily than a particular active ingredient and therefore can scavenge oxygen, e.g., ascorbic acid and sulfites; (ii) chain terminators, which are molecules that form stable radicals due to weak bonds to hydrogen atoms that are attacked in a propagation of radical chains by consumption of oxygen, e.g., methionine, NAC, glutathionine, lipoic acid, butylated hydroxytoluene (BHT), and cysteine, (iii) chelating agents, which reduce catalytic activity of transition metals by forming complexes with the metals, e.g., EDTA, EGTA and DTPA and analogs thereof.

"Biological activity" describes the beneficial or adverse effects of a drug on living matter. When a drug is a complex chemical mixture, this activity is exerted by the substance's active ingredient but can be modified by the other constituents. Biological activity can be assessed as potency or as toxicity by an *in vivo* LD₅₀ or ED₅₀ assay, or through an *in vitro* assay such as, for example, cell-based potency assays (CBPAs) as described in U.S. 20100203559 and U.S. 20100233802. The activities of the compositions of the invention may be measured using any sutiable assay and are not limited to activities measured by CBPA. The potency described in the examples also encompasses potency when measured using LD50.

"Botulinum toxin" means a neurotoxin produced by Clostridium botulinum, as well as a botulinum toxin (or the light chain or the heavy chain thereof) made recombinantly by a non-Clostridial species. The phrase "botulinum toxin", as used herein, encompasses the botulinum toxin serotypes A, B, C, D, E, F and G, and their subtypes and any other types of subtypes thereof, or any re-engineered proteins, analogs, derivatives, homologs, parts, subparts, variants, or versions, in each case, of any of the foregoing. "Botulinum toxin", as used herein, also encompasses a "modified botulinum toxin". Further "botulinum toxin" as used herein also encompasses a botulinum toxin complex, (for example, the 300, 600 and 900kDa complexes), as well as the neurotoxic component of the botulinum toxin (150 kDa) that is unassociated with the complex proteins.

"Clostridial toxin" refers to any toxin produced by a Clostridial toxin strain that can execute the overall cellular mechanism whereby a Clostridial toxin intoxicates a cell and encompasses the binding of a Clostridial toxin to a low or high affinity Clostridial toxin receptor, the internalization of the toxin/receptor complex, the translocation of the Clostridial toxin light chain into the cytoplasm and the enzymatic modification of a Clostridial toxin substrate. Non-limiting examples of Clostridial toxins include a Botulinum toxin like BoNT/A, a BoNT/B, a BoNT/C₁, a BoNT/D, a BoNT/E, a BoNT/F, a BoNT/G, a Tetanus toxin (TeNT), a Baratii toxin (BaNT), and a Butyricum toxin (BuNT). The BoNT/C₂ cytotoxin and BoNT/C₃ cytotoxin, not being neurotoxins, are excluded from the term "Clostridial toxin." A Clostridial toxin disclosed herein includes, without limitation, naturally occurring Clostridial toxin variants, such as, *e.g.,* Clostridial toxin isoforms and Clostridial toxin subtypes; non-naturally occurring Clostridial toxin variants, such as, *e.g.,* conservative Clostridial toxin variants, non-conservative Clostridial toxin variants, Clostridial toxin chimeric variants and active Clostridial toxin fragments thereof, or any combination thereof. A Clostridial toxin disclosed herein also includes a Clostridial toxin complex. As used herein, the term "Clostridial toxin complex" refers to a complex comprising a Clostridial toxin and non-toxin associated proteins (NAPs), such as, *e.g.,* a Botulinum toxin complex, a Tetanus toxin complex, a Baratii toxin complex, and a Butyricum toxin complex. Non-limiting examples of Clostridial toxin complexes include those produced by a *Clostridium botulinum,* such as, *e.g.,* a 900-kDa BoNT/A complex, a 500-kDa BoNT/A complex, a 300-kDa BoNT/A complex, a 500-kDa BoNT/B complex, a 500-kDa BoNT/C₁ complex, a 500-kDa BoNT/D complex, a 300-kDa BoNT/D complex, a 300-kDa BoNT/E complex, and a 300-kDa BoNT/F complex.

"Clostridial toxin active ingredient" refers to a molecule which contains any part of a clostridial toxin that exerts an effect upon or after administration to a subject or patient. As used herein, the term "clostridial toxin active ingredient" encompasses a Clostridial toxin complex comprising the approximately 150-kDa Clostridial toxin and other proteins collectively called non-toxin associated proteins (NAPs), the approximately 150-kDa Clostridial toxin alone, or a modified Clostridial toxin, such as, *e.g.,* a re-targeted Clostridial toxins.

"Deformity" means a cosmetic, physical or functional irregularity, defect, abnormality, imperfection, malformation, depression, or distortion.

"Effective amount" as applied to the biologically active ingredient means that amount of the ingredient which is generally sufficient to effect a desired change in the subject. For example, where the desired effect is a reduction in an autoimmune disorder symptom, an effective amount of the ingredient is that amount which causes at least a substantial reduction of the autoimmune disorder symptom, and without resulting in significant toxicity.

"Effective amount" when used in reference to the amount of an excipient or specific combination of excipients added to a Clostridial toxin composition, refers to the amount of each excipient that is necessary to achieve the desired initial recovered potency of a Clostridial toxin active ingredient. In aspects of this embodiment, an effective amount of an excipient or combination of excipients results in an initial recovered potency of, *e.g.,* at least 10%, at least 20%, at least 30%, at least 40%, at least 50%, at least 60%, at least 70%, at least 80%, at least 90% or at least 100%. In other aspects of this embodiment, a therapeutically effective concentration of a Clostridial toxin active ingredient reduces a symptom associated with the aliment being treated by, *e.g.,* at most 10%, at most 20%,at most 30%, at most 40%, at most 50%, at most 60%, at most 70%, at most 80%, at most 90% or at most 100%.

"Heavy chain" means the heavy chain of a botulinum neurotoxin. It has a molecular weight of about 100kDa and can be referred to as the H chain, or as H.

H_{C} means a fragment (about 50kDa) derived from the H chain of a botulinum neurotoxin which is approximately equivalent to the carboxyl end segment of the H chain, or the portion corresponding to that fragment in the intact H chain. It is believed to be immunogenic and to contain the portion of the natural or wild type botulinum neurotoxin involved in high affinity, presynaptic binding to motor neurons.

H_{N} means a fragment (about 50kDa) derived from the H chain of a botulinum neurotoxin which is approximately equivalent to the amino end segment of the H chain, or the portion corresponding to that fragment in the intact in the H chain. It is believed to contain the portion of the natural or wild type botulinum neurotoxin involved in the translocation of the L chain across an intracellular endosomal membrane.

"Light chain" means the light chain of a clostridial neurotoxin. It has a molecular weight of about 50kDa, and can be referred to as the L chain, L, or as the proteolytic domain (amino acid sequence) of a botulinum neurotoxin.

LH_{N} or L-H_{N} means a fragment derived from a clostridial neurotoxin that contains the L chain, or a functional fragment thereof coupled to the H_{N} domain It can be obtained from the intact clostridial neurotoxin by proteolysis, so as to remove or to modify the H_{C} domain.

"Implant" means a controlled release (e.g., pulsatile or continuous) composition or drug delivery system. The implant can be, for example, injected, inserted or implanted into a human body.

"Liquid composition", "liquid pharmaceutical composition", or "liquid formulation" refers to a pharmaceutically active preparation of drug or biologically active ingredient which is capable of being stored in a liquid pharmaceutical excipient, such as a buffering agent, for an extended period of time, such that it can be ready-to-use as needed by a clinician. The liquid pharmaceutical composition is manufactured without a lyophilization process.

"Local administration" means direct administration of a pharmaceutical at or to the vicinity of a site on or within an animal body, at which site a biological effect of the pharmaceutical is desired, such as via, for example, intramuscular or intra- or subdermal injection or topical administration. Local administration excludes systemic routes of administration, such as intravenous or oral administration. Topical administration is a type of local administration in which a pharmaceutical agent is applied to a patient's skin.

"Lyoprotector" or "lyoprotectant" means a substance that is included in a lyophilized formulation to protect a Clostridial toxin active ingredient during the freeze-drying process. Lyoprotectors include for example polyhydroxy compounds such as sugars (mono-, di-, and polysaccharides), polyalcohols, and their derivatives. Exemplary lyoprotectors which can be used with the lyophilized formulations disclosed herein include sucrose, trehalose, mannitol, sorbitol, glucose, raffinose, maltose, glycerol, lactose, fructose, galactose, and combinations thereof.

"Lyophilized composition", "lyophilized pharmaceutical composition", "lyophilized formulation", or "solid composition" refers to a formulation containing a Clostridial toxin active ingredient which has been subjected to a lyophilization, freeze-drying or vacuum-drying process; and can be reconstituted with a reconstitution vehicle, such as for example saline or water, prior to administration to a patient. The lyophilized composition can be a freeze-dried composition or a vacuum-dried composition.

"Modified botulinum toxin" means a botulinum toxin that has had at least one of its amino acids deleted, modified, or replaced, as compared to a native botulinum toxin. Additionally, the modified botulinum toxin can be a recombinantly produced neurotoxin, or a derivative or fragment of a recombinantly made neurotoxin. A modified botulinum toxin retains at least one biological activity of the native botulinum toxin, such as, the ability to bind to a botulinum toxin receptor, or the ability to inhibit neurotransmitter release from a neuron. One example of a modified botulinum toxin is a botulinum toxin that has a light chain from one botulinum toxin serotype (such as serotype A), and a heavy chain from a different botulinum toxin serotype (such as serotype B). Another example of a modified botulinum toxin is a botulinum toxin coupled to a neurotransmitter, such as substance P.

"Mutation" means a structural modification of a naturally occurring protein or nucleic acid sequence. For example, in the case of nucleic acid mutations, a mutation can be a deletion, addition or substitution of one or more nucleotides in the DNA sequence. In the case of a protein sequence mutation, the mutation can be a deletion, addition or substitution of one or more amino acids in a protein sequence. For example, a specific amino acid comprising a protein sequence can be substituted for another amino acid, for example, an amino acid selected from a group which includes the amino acids alanine, aspargine, cysteine, aspartic acid, glutamic acid, phenylalanine, glycine, histidine, isoleucine, lysine, leucine, methionine, proline, glutamine, arginine, serine, threonine, valine, tryptophan, tyrosine or any other natural or non-naturally occurring amino acid or chemically modified amino acids. Mutations to a protein sequence can be the result of mutations to DNA sequences that when transcribed, and the resulting mRNA translated, produce the mutated protein sequence. Mutations to a protein sequence can also be created by fusing a peptide sequence containing the desired mutation to a desired protein sequence.

"Patient" means a human or non-human subject receiving medical or veterinary care. Accordingly, the compositions as disclosed herein can be used in treating any animal, such as, for example, mammals, or the like.

"Peripherally administering" or "peripheral administration" means subdermal, intradermal, transdermal, or subcutaneous administration, but excludes intramuscular administration. "Peripheral" means in a subdermal location, and excludes visceral sites.

"Pharmaceutical composition" means a composition comprising an active pharmaceutical ingredient, such as, for example, a clostridial toxin active ingredient such as a botulinum toxin, and at least one additional ingredient, such as, for example, a stabilizer or excipient or the like. A pharmaceutical composition is therefore a formulation which is suitable for diagnostic or therapeutic administration to a subject, such as a human patient. The pharmaceutical composition can be, for example, in a lyophilized or vacuum dried condition, a solution formed after reconstitution of the lyophilized or vacuum dried pharmaceutical composition, or as a solution or solid which does not require reconstitution.

"Pharmacologically acceptable excipient" is synonymous with "pharmacological excipient" or "excipient" and refers to any excipient that has substantially no long term or permanent detrimental effect when administered to mammal and encompasses compounds such as, *e.g.,* stabilizing agent, a bulking agent, a cryo-protectant, a lyo-protectant, an additive, a vehicle, a carrier, a diluent, or an auxiliary. An excipient generally is mixed with an active ingredient, or permitted to dilute or enclose the active ingredient and can be a solid, semi-solid, or liquid agent. It is also envisioned that a pharmaceutical composition comprising a Clostridial toxin active ingredient can include one or more pharmaceutically acceptable excipients that facilitate processing of an active ingredient into pharmaceutically acceptable compositions. Insofar as any pharmacologically acceptable excipient is not incompatible with the Clostridial toxin active ingredient, its use in pharmaceutically acceptable compositions is contemplated. Non-limiting examples of pharmacologically acceptable excipients can be found in, *e.g.,* Pharmaceutical Dosage Forms and Drug Delivery Systems (Howard C. Ansel et al., eds., Lippincott Williams & Wilkins Publishers, 7th ed. 1999); Remington: The Science and Practice of Pharmacy (Alfonso R. Gennaro ed., Lippincott, Williams & Wilkins, 20th ed. 2000); Goodman & Gilman's The Pharmacological Basis of Therapeutics (Joel G. Hardman et al., eds., McGraw-Hill Professional, 10th ed. 2001); and Handbook of Pharmaceutical Excipients (Raymond C. Rowe et al., APhA Publications, 4th edition 2003), each of which is hereby incorporated by reference in its entirety.

The constituent ingredients of a pharmaceutical composition can be included in a single composition (that is, all the constituent ingredients, except for any required reconstitution fluid, are present at the time of initial compounding of the pharmaceutical composition) or as a two-component system, for example a vacuum-dried composition reconstituted with a reconstitution vehicle which can, for example, contain an ingredient not present in the initial compounding of the pharmaceutical composition. A two-component system can provide several benefits, including that of allowing incorporation of ingredients which are not sufficiently compatible for long-term shelf storage with the first component of the two component system. For example, the reconstitution vehicle may include a preservative which provides sufficient protection against microbial growth for the use period, for example one-week of refrigerated storage, but is not present during the two-year freezer storage period during which time it might degrade the toxin. Other ingredients, which may not be compatible with a botulinum toxin or other ingredients for long periods of time, can be incorporated in this manner; that is, added in a second vehicle (*e.g.* in the reconstitution vehicle) at the approximate time of use. A pharmaceutical composition can also include preservative agents such as benzyl alcohol, benzoic acid, phenol, parabens and sorbic acid. Pharmaceutical compositions can include, for example, excipients, such as surface active agents; dispersing agents; inert diluents; granulating and disintegrating agents; binding agents; lubricating agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; antioxidants; stabilizing agents; and pharmaceutically acceptable polymeric or hydrophobic materials and other ingredients known in the art and described, for example in Genaro, ed., 1985, Remington's Pharmaceutical Sciences, Mack Publishing Co., Easton, Pa., which is incorporated herein by reference.

"Polysaccharide" means a polymer of more than two saccharide molecule monomers. The monomers can be identical or different.

"Stabilizing agent", "stabilization agent" or "stabilizer" means a substance that acts to stabilize a Clostridial toxin active ingredient such that the potency of the pharmaceutical composition is increased relative to an unstabilized composition.

"Stabilizers" can include excipients, and can include protein and non-protein molecules.

"Surfactant" refers to a natural or synthetic amphiphilic compound. A surfactant can be non-ionic, zwitterionic, or ionic. Non-limiting examples of surfactants include a poloxamer, a polysorbate, and combinations thereof.

"Therapeutic formulation" means a formulation can be used to treat and thereby alleviate a disorder or a disease, such as, for example, a disorder or a disease characterized by hyperactivity (*i.e.* spasticity) of a peripheral muscle.

"TEM" as used herein, is synonymous with "Targeted Exocytosis Modulator" or "retargeted endopeptidase." Generally, a TEM comprises an enzymatic domain from a Clostridial toxin light chain, a translocation domain from a Clostridial toxin heavy chain, and a targeting domain. The targeting domain of a TEM provides an altered cell targeting capability that targets the molecule to a receptor other than the native Clostridial toxin receptor utilized by a naturally-occurring Clostridial toxin. This re-targeted capability is achieved by replacing the naturally-occurring binding domain of a Clostridial toxin with a targeting domain having a binding activity for a non-Clostridial toxin receptor. Although binding to a non-Clostridial toxin receptor, a TEM undergoes all the other steps of the intoxication process including internalization of the TEM/receptor complex into the cytoplasm, formation of the pore in the vesicle membrane and di-chain molecule, translocation of the enzymatic domain into the cytoplasm, and exerting a proteolytic effect on a component of the SNARE complex of the target cell.

"Tonicity agent" or "isotonic agent" means a low molecular weight excipient which is included in a lyophilized or liquid formulation to provide isotonicity. For example, a disaccharide such as trehalose or sucrose, a polyalcohol such as sorbitol or mannitol, a monosaccharide such as glucose, and a salt such as sodium chloride, can serve as a tonicity agent.

"Topical administration" excludes systemic administration of the neurotoxin. In other words, and unlike conventional therapeutic transdermal methods, topical administration of botulinum toxin does not result in significant amounts, such as the majority of, the neurotoxin passing into the circulatory system of the patient.

"Treating" means to alleviate (or to eliminate) at least one symptom of a condition or disorder, such as, for example, wrinkles, spasticity, depression, pain (such as, for example, headache pain), bladder overactivity, or the like, either temporarily or permanently.

As used herein, the term "unit" or "U" refers to the LD₅₀ dose or the dose determined by a cell based potency assay (CBPA). The LD₅₀ dose is defined as the amount of a Clostridial toxin active ingredient, Clostridial toxin complex or modified Clostridial toxin that killed 50% of the mice injected with the Clostridial toxin, Clostridial toxin complex or modified Clostridial toxin. The CBPA dose is determined as described in US Patent No. 8,618,261, the assay details of which are incorporated by reference herein.

"Variant" means a clostridial neurotoxin, such as wild-type botulinum toxin serotype A, B, C, D, E, F or G, that has been modified by the replacement, modification, addition or deletion of at least one amino acid relative to wild-type botulinum toxin, which is recognized by a target cell, internalized by the target cell, and catalytically cleaves a SNARE (SNAP (Soluble NSF Attachment Protein) Receptor) protein in the target cell.

An example of a variant neurotoxin component can comprise a variant light chain of a botulinum toxin having one or more amino acids substituted, modified, deleted and/or added. This variant light chain may have the same or better ability to prevent exocytosis, for example, the release of neurotransmitter vesicles. Additionally, the biological effect of a variant may be decreased compared to the parent chemical entity. For example, a variant light chain of a botulinum toxin type A having an amino acid sequence removed may have a shorter biological persistence than that of the parent (or native) botulinum toxin type A light chain.

In one aspect, the composition does not comprise a sugar or polyalcohol. Sugar is defined in the next section. As used herein, the term "polyalcohol" is synonymous with "sugar alcohol," "polyhydric alcohol," and "polyol" and refers to a sugar derivative having an alcohol group (CH₂OH) instead of the aldehyde group (CHO), such as, e.g., mannitol from mannose, xylitol from xylose, and lactitol from lactulose. Non-limiting examples of polyols include, glycol, glycerol, arabitol, erythritol, xylitol, maltitol, sorbitol (gluctiol), mannitol, inositol, lactitol, galactitol (iditol), isomalt. Other non-limiting examples of sugar excipients can be found in, e.g., Ansel, supra, (1999); Gennaro, supra, (2000); Hardman, supra, (2001); and Rowe, supra, (2003), each of which is hereby incorporated by reference in its entirety.

### Pharmaceutical compositions

Certain embodiments of the present invention include a pharmaceutical composition comprising (or consisting of, or consisting essentially of) a Clostridial toxin active ingredient such as a botulinum toxin, a disaccharide, a surfactant and an antioxidant.

Aspects of the present pharmaceutical compositions provide, in part, a Clostridial toxin active ingredient. As used herein, the term "Clostridial toxin active ingredient" refers to a therapeutically effective concentration of a Clostridial toxin active ingredient, such as, *e.g.*, a Clostridial toxin complex, a Clostridial toxin, a modified Clostridial toxin, or a re-targeted Clostridial toxin. A botulinum toxin is a particularly preferred Clostridial toxin active ingredient for use in the invention. As used herein, the term "therapeutically effective concentration" is synonymous with "therapeutically effective amount," "effective amount," "effective dose," and "therapeutically effective dose" and refers to the minimum dose of a Clostridial toxin active ingredient, which is most preferably a botulinum toxin, necessary to achieve the desired therapeutic effect and includes a dose sufficient to reduce a symptom associated with aliment being treated. In aspects of this embodiment, a therapeutically effective concentration of a Clostridial toxin active ingredient, which is most preferably a botulinum toxin, reduces a symptom associated with the aliment being treated by, *e.g.,* at least 10%, at least 20%, at least 30%, at least 40%, at least 50%, at least 60%, at least 70%, at least 80%, at least 90% or at least 100%. In other aspects of this embodiment, a therapeutically effective concentration of a Clostridial toxin active ingredient, which is most preferably a botulinum toxin, reduces a symptom associated with the aliment being treated by, *e.g.,* at most 10%, at most 20%, at most 30%, at most 40%, at most 50%, at most 60%, at most 70%, at most 80%, at most 90% or at most 100%.

It is envisioned that any amount of Clostridial toxin active ingredient, which is most preferably a botulinum toxin, can be added in formulating a Clostridial toxin pharmaceutical compositions disclosed in the present specification, with the proviso that a therapeutically effective amount of the Clostridial toxin active ingredient is recoverable. In aspects of this embodiment, the amount of Clostridial toxin active ingredient, which is most preferably a botulinum toxin, added to the formulation is at least 0.1 U/ml, at least 1.0 U/ml, at least 10 U/ml, at least 50 U/ml, at least 100 U/ml, at least 200 U/ml, or at least 1000 U/ml. In other aspects of this embodiment, the amount of Clostridial toxin active ingredient, which is most preferably a botulinum toxin, added to the formulation is at most 0.1 U/ml, at most 1.0 U/ml, at most 10 U/ml, at most 50 U/ml, at most 100 U/ml, at most 200 U/ml, or at most 1000 U/ml. In yet other aspects of this embodiment, the amount of Clostridial toxin active ingredient, which is most preferably a botulinum toxin, added to the formulation is from about 0.1 U/ml to about 1000 U/ml, or about 1.0 U/ml to about 1000 U/ml. In still other aspects of this embodiment, the amount of Clostridial toxin active ingredient, which is most preferably a botulinum toxin, added to the formulation is from about 0.001 U/ml to about 100 U/ml, about 0.01 U/ml to about 100 U/ml, about 0.1 U/ml to about 100 U/ml, or about 1.0 U/ml to about 100 U/ml.

In other aspects of this embodiment, the amount of Clostridial toxin active ingredient, which is most preferably a botulinum toxin, added to the formulation is at least 1.0 pg, at least 10 pg, at least 100 pg, at least 1.0 ng, at least 10 ng, at least 100 ng, at least 1.0 µg, at least 10 µg, at least 100 µg, or at least 1.0 mg. In still other aspects of this embodiment, the amount of Clostridial toxin active ingredient, which is most preferably a botulinum toxin, added to the formulation is at most 1.0 pg, at most 10 pg, at most 100 pg, at most 1.0 ng, at most 10 ng, at most 100 ng, at most 1.0 µg, at most 10 µg, at most 100 µg, or at most 1.0 mg. In still other aspects of this embodiment, the amount of Clostridial toxin active ingredient, which is most preferably a botulinum toxin, added to the formulation is about 1.0 pg to about 10 µg, about 10 pg to about 10 µg, about 100 pg to about 10 µg, about 1.0 ng to about 10 µg, about 10 ng to about 10 µg, or about 100 ng to about 10 µg. In still other aspects of this embodiment, the amount of Clostridial toxin active ingredient, which is most preferably a botulinum toxin, added to the formulation is about 1.0 pg to about 1.0 µg, about 10 pg to about 1.0 µg, about 100 pg to about 1.0 µg, about 1.0 ng to about 1.0 µg, about 10 ng to about 1.0 µg, or about 100 ng to about 1.0 µg. In further aspects of this embodiment, the amount of Clostridial toxin active ingredient, which is most preferably a botulinum toxin, added to the formulation is about 1.0 pg to about 5.0 µg, about 10 pg to about 5.0 µg, about 100 pg to about 5.0 µg, about 1.0 ng to about 5.0 µg, about 10 ng to about 5.0 µg, or about 100 ng to about 5.0 µg. In further aspects of this embodiment, the amount of Clostridial toxin active ingredient, which is most preferably a botulinum toxin, added to the formulation is about 1.0 pg to about 10 µg, about 10 pg to about 10 µg, about 100 pg to about 10 µg, about 1.0 ng to about 10 µg, about 10 ng to about 10 µg, or about 100 ng to about 10 µg.

In aspects of this embodiment, a Clostridial toxin pharmaceutical composition comprises a BoNT/A, a BoNT/B, a BoNT/C₁, a BoNT/D, a BoNT/E, a BoNT/F, a BoNT/G, a BoNT mosaic, such as BoNT/DC, BoNT/CD and BoNT/FA, a TeNT, a BaNT, or a BuNT. In another embodiment, a Clostridial toxin pharmaceutical composition comprises a Clostridial toxin variant as the Clostridial toxin active ingredient. In aspects of this embodiment, a Clostridial toxin pharmaceutical composition comprises naturally-occurring Clostridial toxin variant or a non-naturally-occurring Clostridial toxin variant. In other aspects of this embodiment, a Clostridial toxin pharmaceutical composition comprises a BoNT/A variant, a BoNT/B variant, a BoNT/C₁ variant, a BoNT/D variant, a BoNT/E variant, a BoNT/F variant, a BoNT/G variant, a TeNT variant, a BaNT variant, or a BuNT variant, where the variant is either a naturally-occurring variant or a non-naturally-occurring variant.

Aspects of the present pharmaceutical compositions provide, in part, a Clostridial toxin complex as a Clostridial toxin active ingredient. As used herein, the term "Clostridial toxin complex" refers to a complex comprising a Clostridial toxin and associated NAPs, such as, *e.g.,* a Botulinum toxin complex, a Tetanus toxin complex, a Baratii toxin complex, and a Butyricum toxin complex. Non-limiting examples of Clostridial toxin complexes include those produced by a *Clostridium botulinum*, such as, *e.g.,* a 900-kDa BoNT/A complex, a 500-kDa BoNT/A complex, a 300-kDa BoNT/A complex, a 500-kDa BoNT/B complex, a 500-kDa BoNT/C₁ complex, a 500-kDa BoNT/D complex, a 300-kDa BoNT/D complex, a 300-kDa BoNT/E complex, and a 300-kDa BoNT/F complex. Clostridial toxin complexes can be purified using the methods described in Schantz, *supra,* (1992); Hui Xiang et al., Animal Product Free System and Process for Purifying a Botulinum Toxin, U.S. Patent 7,354,740, each of which is hereby incorporated by reference in its entirety. Clostridial toxin complexes can be obtained from, *e.g.*, List Biological Laboratories, Inc. (Campbell, California), the Centre for Applied Microbiology and Research (Porton Down, U.K), Wako (Osaka, Japan), and Sigma Chemicals (St Louis, Missouri).

Aspects of the present pharmaceutical compositions provide, in part, a non-protein excipient. As used herein, the term "non-protein excipient" refers to any excipient that is not a polypeptide comprising at least fifteen amino acids. It is envisioned that any non-protein excipient is useful in formulating a Clostridial toxin pharmaceutical compositions disclosed in the present specification, with the proviso that a therapeutically effective amount of the Clostridial toxin active ingredient, which is most preferably a botulinum toxin, is recovered using this non-protein excipient.

Aspects of the present pharmaceutical compositions provide, in part, a sugar. As used herein, the term "sugar" refers to a compound comprising one to 10 monosaccharide units, *e.g.,* a monosaccharide, a disaccharide, a trisaccharide, and an oligosaccharide comprising four to ten monosaccharide units. It is envisioned that any sugar is useful in formulating a Clostridial toxin pharmaceutical compositions disclosed in the present specification, with the proviso that a therapeutically effective amount of the Clostridial toxin active ingredient, which is most preferably a botulinum toxin, is recovered using this sugar. In some embodiments, for example in a lyophilized composition, the sugar can function as a lyoprotector. In some other embodiments, for example in a lyophilized formulation or in a liquid formulation, the sugar can function as a tonicity agent. Monosaccharides are polyhydroxy aldehydes or polyhydroxy ketones with three or more carbon atoms, including aldoses, dialdoses, aldoketoses, ketoses and diketoses, as well as cyclic forms, deoxy sugars and amino sugars, and their derivatives, provided that the parent monosaccharide has a (potential) carbonyl group. Monosacchrides include trioses, like glyceraldehyde and dihydroxyacetone; tetroses, like erythrose, erythrulose and threose; pentoses, like arabinose, lyxose, ribose, ribulose, xylose, xylulose; hexoses, like allose, altrose, fructose, fucose,galactose, glucose, gulose, idose, mannose, psicose, rhamnose, sorbose, tagatose, talose and trehalose; heptoses, like sedoheptulose and mannoheptulose; octooses, like octulose and 2-keto-3-deoxy-manno-octonate; nonoses like sialose; and decose. Oligo saccharides are compounds in which at least two monosaccharide units are joined by glycosidic linkages. According to the number of units, they are called disaccharides, trisaccharides, tetrasaccharides, pentasaccharides, hexoaccharides, heptoaccharides, octoaccharides, nonoaccharides, decoaccharides, etc. An oligosaccharide can be unbranched, branched or cyclic. Common disaccharides include, without limitation, sucrose, lactose, maltose, trehalose, cellobiose, gentiobiose, kojibiose, laminaribiose, mannobiose, melibiose, nigerose, rutinose, and xylobiose. Common trisaccharides include, without limitation, raffinose, acarbose, maltotriose, and melezitose. Other non-limiting examples of specific uses of sugar excipients can be found in, *e.g.,* Ansel, *supra,* (1999); Gennaro, *supra*, (2000); Hardman, *supra,* (2001); and Rowe, *supra,* (2003), each of which is hereby incorporated by reference in its entirety.

In an embodiment, a Clostridial toxin pharmaceutical composition comprises a sugar. In aspects of this embodiment, a Clostridial toxin pharmaceutical composition comprises a monosaccharide. In other aspects of this embodiment, a Clostridial toxin pharmaceutical composition comprises a disaccharide, a trisaccharide, a tetrasaccharide, a pentasaccharide, a hexoaccharide, a heptoaccharide, an octoaccharide, a nonoaccharide, or a decoaccharide. In yet other aspects of this embodiment, a Clostridial toxin pharmaceutical composition comprises an oligosaccharide comprising two to ten monosaccharide units. Trehalose and sucrose are particularly preferred sugars for use in the invention.

It is envisioned that any amount of sugar, which is preferably trehalose or sucrose, is useful in formulating a Clostridial toxin pharmaceutical compositions disclosed in the present specification, with the proviso that a therapeutically effective amount of the Clostridial toxin active ingredient, which is most preferably a botulinum toxin, is recovered using this sugar amount. In aspects of this embodiment, the amount of sugar, which is preferably trehalose or sucrose, added to the formulation is about 0.1% (w/w), about 0.5% (w/w), about 1.0% (w/w), about 1.5% (w/w), about 2.0% (w/w), about 2.5% (w/w), about 3.0% (w/w), about 3.5% (w/w), about 4.0% (w/w), about 4.5% (w/w), about 5.0% (w/w), about 5.5% (w/w), about 6.0% (w/w), about 6.5% (w/w), about 7.0% (w/w), about 7.5% (w/w), about 8.0% (w/w), about 8.5% (w/w), about 9.0% (w/w), about 9.5% (w/w), about 10% (w/w), about 15% (w/w), about 20% (w/w), about 25% (w/w), about 30% (w/w), or about 35% (w/w). In other aspects of this embodiment, the amount of sugar, which is preferably trehalose or sucrose, added to the formulation is at least 0.1% (w/w), at least 0.5% (w/w), at least 1.0% (w/w), at least 1.5% (w/w), at least 2.0% (w/w), at least 2.5% (w/w), at least 3.0% (w/w), at least 3.5% (w/w), at least 4.0% (w/w), at least 4.5% (w/w), at least 5.0% (w/w), at least 5.5% (w/w), at least 6.0% (w/w), at least 6.5% (w/w), at least 7.0% (w/w), at least 7.5% (w/w), at least 8.0% (w/w), at least 8.5% (w/w), at least 9.0% (w/w), at least 9.5% (w/w), at least 10% (w/w), at least 15% (w/w), at least 20% (w/w), at least 25% (w/w), at least 30% (w/w), or at least 35% (w/w). In yet other aspects of this embodiment, the amount of sugar, which is preferably trehalose or sucrose, added to the formulation is at most 0.1% (w/w), at most 0.5% (w/w), at most 1.0% (w/w), at most 1.5% (w/w), at most 2.0% (w/w), at most 2.5% (w/w), at most 3.0% (w/w), at most 3.5% (w/w), at most 4.0% (w/w), at most 4.5% (w/w), at most 5.0% (w/w), at most 5.5% (w/w), at most 6.0% (w/w), at most 6.5% (w/w), at most 7.0% (w/w), at most 7.5% (w/w), at most 8.0% (w/w), at most 8.5% (w/w), at most 9.0% (w/w), at most 9.5% (w/w), at most 10% (w/w), at most 15% (w/w), at most 20% (w/w), at most 25% (w/w), at most 30% (w/w), or at most 35% (w/w).

In an embodiment, the present Clostridial toxin pharmaceutical composition comprises a disaccharide, which is preferably trehalose or sucrose. Common disaccharides include, without limitation, sucrose, lactose, maltose, trehalose, cellobiose, gentiobiose, kojibiose, laminaribiose, mannobiose, melibiose, nigerose, rutinose, and xylobiose. In aspects of this embodiment, the clostridial toxin pharmaceutical composition comprises sucrose. In one specific embodiment, the clostridial toxin pharmaceutical composition comprises trehalose. In aspects of this embodiment, the amount of disaccharide, which is preferably trehalose or sucrose, added to the formulation added to the formulation is about 0.1% (w/w), about 0.5% (w/w), about 1.0% (w/w), about 1.5% (w/w), about 2.0% (w/w), about 2.5% (w/w), about 3.0% (w/w), about 3.5% (w/w), about 4.0% (w/w), about 4.5% (w/w), about 5.0% (w/w), about 5.5% (w/w), about 6.0% (w/w), about 6.5% (w/w), about 7.0% (w/w), about 7.5% (w/w), about 8.0% (w/w), about 8.5% (w/w), about 9.0% (w/w), about 9.5% (w/w), about 10% (w/w), about 15% (w/w), about 20% (w/w), about 25% (w/w), about 30% (w/w), or about 35% (w/w).

Aspects of the present pharmaceutical compositions provide, in part, a surfactant. As used hereon, the term "surfactant" refers to a natural or synthetic amphiphilic compound. A surfactant can be non-ionic, zwitterionic, or ionic. It is envisioned that any surfactant is useful in formulating a Clostridial toxin pharmaceutical compositions disclosed in the present specification, with the proviso that a therapeutically effective amount of the Clostridial toxin active ingredient, which is most preferably a botulinum toxin, is recovered using this surfactant amount. Non-limiting examples of surfactants include polysorbates like polysorbate 20 (TWEEN^{®} 20), polysorbate 40 (TWEEN^{®} 40), polysorbate 60 (TWEEN^{®} 60), polysorbate 61 (TWEEN^{®} 61), polysorbate 65 (TWEEN^{®} 65), polysorbate 80 (TWEEN^{®} 80), and polysorbate 81 (TWEEN^{®} 81); poloxamers (polyethylene-polypropylene copolymers), like Poloxamer 124 (PLURONIC^{®} L44), Poloxamer 181 (PLURONIC^{®} L61), Poloxamer 182 (PLURONIC^{®} L62), Poloxamer 184 (PLURONIC^{®} L64), Poloxamer 188 (PLURONIC^{®} F68), Poloxamer 237 (PLURONIC^{®} F87), Poloxamer 338 (PLURONIC^{®} L108), Poloxamer 407 (PLURONIC^{®} F127), polyoxyethyleneglycol dodecyl ethers, like BRIJ^{®} 30, and BRIJ^{®} 35; 2-dodecoxyethanol (LUBROL^{®}-PX); polyoxyethylene octyl phenyl ether (TRITON^{®} X-100); sodium dodecyl sulfate (SDS); solutol HS15; 3-[(3-Cholamidopropyl)dimethylammonio]-1-propanesulfonate (CHAPS); 3-[(3-Cholamidopropyl)dimethylammonio]-2-hydroxy-1-propanesulfonate (CHAPSO); sucrose monolaurate; and sodium cholate. Other non-limiting examples of surfactant excipients can be found in, *e.g.,* Ansel, *supra,* (1999); Gennaro, *supra,* (2000); Hardman, *supra,* (2001); and Rowe, *supra,* (2003), each of which is hereby incorporated by reference in its entirety. Poloxamer 188 is a particularly preferred surfactant in the invention.

Thus in an embodiment, a Clostridial toxin pharmaceutical composition comprises a surfactant, which is preferably Poloxamer 188. In aspects of this embodiment, a Clostridial toxin pharmaceutical composition comprises a polysorbate, a poloxamer, a polyoxyethyleneglycol dodecyl ether, 2-dodecoxyethanol , polyoxyethylene octyl phenyl ether, sodium dodecyl sulfate, 3-[(3-Cholamidopropyl)dimethylammonio]-1-propanesulfonate, 3-[(3-Cholamidopropyl) dimethylammonio]-2-hydroxy-1-propanesulfonate, sucrose monolaurate; or sodium cholate.

It is envisioned that any amount of surfactant, which is preferably Poloxamer 188, is useful in formulating a Clostridial toxin pharmaceutical compositions disclosed in the present specification, with the proviso that a therapeutically effective amount of the Clostridial toxin active ingredient, which is preferably a botulinum toxin, is recovered using this surfactant amount. In aspects of this embodiment, the amount of surfactant, which is preferably Poloxamer 188, added to the formulation is about 0.01% (w/w), about 0.02% (w/w), about 0.03% (w/w), about 0.04% (w/w), about 0.05% (w/w), about 0.06% (w/w), about 0.07% (w/w), about 0.08% (w/w), about 0.09% (w/w), about 0.1% (w/w), about 0.5% (w/w), about 1.0% (w/w), about 1.5% (w/w), about 2.0% (w/w), about 2.5% (w/w), about 3.0% (w/w), about 3.5% (w/w), about 4.0% (w/w), about 4.5% (w/w), about 5.0% (w/w), about 5.5% (w/w), about 6.0% (w/w), about 6.5% (w/w), about 7.0% (w/w), about 7.5% (w/w), about 8.0% (w/w), about 8.5% (w/w), about 9.0% (w/w), about 9.5% (w/w), about 10% (w/w), about 15% (w/w), about 20% (w/w), about 25% (w/w), about 30% (w/w), or about 35% (w/w). In other aspects of this embodiment, the amount of surfactant, which is preferably Poloxamer 188, added to the formulation is at least 0.01% (w/w), at least 0.02% (w/w), at least 0.03% (w/w), at least 0.04% (w/w), at least 0.05% (w/w), at least 0.06% (w/w), at least 0.07% (w/w), at least 0.08% (w/w), at least 0.09% (w/w), at least 0.1% (w/w), at least 0.5% (w/w), at least 1.0% (w/w), at least 1.5% (w/w), at least 2.0% (w/w), at least 2.5% (w/w), at least 3.0% (w/w), at least 3.5% (w/w), at least 4.0% (w/w), at least 4.5% (w/w), at least 5.0% (w/w), at least 5.5% (w/w), at least 6.0% (w/w), at least 6.5% (w/w), at least 7.0% (w/w), at least 7.5% (w/w), at least 8.0% (w/w), at least 8.5% (w/w), at least 9.0% (w/w), at least 9.5% (w/w), at least 10% (w/w), at least 15% (w/w), at least 20% (w/w), at least 25% (w/w), at least 30% (w/w), or at least 35% (w/w). In yet other aspects of this embodiment, the amount of surfactant, which is preferably Poloxamer 188, added to the formulation is at most 0.01% (w/w), at most 0.02% (w/w), at most 0.03% (w/w), at most 0.04% (w/w), at most 0.05% (w/w), at most 0.06% (w/w), at most 0.07% (w/w), at most 0.08% (w/w), at most 0.09% (w/w), at most 0.1% (w/w), at most 0.5% (w/w), at most 1.0% (w/w), at most 1.5% (w/w), at most 2.0% (w/w), at most 2.5% (w/w), at most 3.0% (w/w), at most 3.5% (w/w), at most 4.0% (w/w), at most 4.5% (w/w), at most 5.0% (w/w), at most 5.5% (w/w), at most 6.0% (w/w), at most 6.5% (w/w), at most 7.0% (w/w), at most 7.5% (w/w), at most 8.0% (w/w), at most 8.5% (w/w), at most 9.0% (w/w), at most 9.5% (w/w), at most 10% (w/w), at most 15% (w/w), at most 20% (w/w), at most 25% (w/w), at most 30% (w/w), or at most 35% (w/w).

In some embodiments, the clostridial toxin pharmaceutical composition comprises a poloxamer, which is preferably Poloxamer 188. Poloxamers which can be used with the present pharmaceutical composition include Poloxamer 124 (PLURONIC^{®} L44), Poloxamer 181 (PLURONIC^{®} L61), Poloxamer 182 (PLURONIC^{®} L62), Poloxamer 184 (PLURONIC^{®} L64), Poloxamer 188 (PLURONIC^{®} F68), Poloxamer 237 (PLURONIC^{®} F87), Poloxamer 338 (PLURONIC^{®} L108), Poloxamer 407 (PLURONIC^{®} F127). In some embodiments, poloxamer 188 may be more advantageous.

In some emobdiments, the clostridial toxin pharmaceutical composition comprises a polysorbate. Polysorbates which can be used with the present pharmaceutical composition includes polysorbate 20 (TWEEN^{®} 20), polysorbate 40 (TWEEN^{®} 40), polysorbate 60 (TWEEN^{®} 60), polysorbate 61 (TWEEN^{®} 61), polysorbate 65 (TWEEN^{®} 65), polysorbate 80 (TWEEN^{®} 80), and polysorbate 81 (TWEEN^{®} 81). In some embodiments, polysorbate 20 may be more advantageous than some other polysorbates.

Aspects of the present pharmaceutical compositions provide, in part, at least an antioxidant. Non-limiting examples of antioxidant include, without limitation, methionine, cysteine, N-acetyl-cysteine (NAC), sodium metabisulfite, sodium thiosulfate, butylated hydroxyanisole, butylated hydroxytoluene, vitamin E and analogs including Trolox C; chelators such as EDTA (ethylene diamine tetraacetic acid sodium salt), EGTA (ethylene glycol-bis(2-aminoethylether)-N,N,N',N'-tetraacetic acid), DTPA (diethylenetriaminepentaacetic acid),analogs or derivatives thereof, and combinations thereof. Methionine and NAC are particularly preferred antioxidants for use in the invention. NAC is particularly preferably used together with EDTA in certain formulations of the invention. In other certain lyophilized formulations of the invention, NAC is the sole antioxidant used. In still other certain formulations of the invention, methionine is the sole antioxidant used. In aspects of these embodiments, the amount of antioxidant, which is preferably methionine or NAC, added to the formulation ranges from about 0.01% (w/w) to about 0.10% (w/w). When used together with NAC, EDTA is preferably added in an amount from about 0.01% (w/w) to about 0.10% (w/w).

It is further envisioned that a Clostridial toxin pharmaceutical composition disclosed in the present specification can optionally include, without limitation, other pharmaceutically acceptable components (or pharmaceutical components), including, without limitation, buffers, preservatives, tonicity adjusters, salts, antioxidants, osmolality adjusting agents, emulsifying agents, sweetening or flavoring agents, and the like. Various buffers and means for adjusting pH can be used to prepare a pharmaceutical composition disclosed in the present specification, provided that the resulting preparation is pharmaceutically acceptable. Such buffers include, without limitation, acetate buffers, borate buffers, citrate buffers, phosphate buffers, neutral buffered saline, and phosphate buffered saline. Histidine is a particularly preferred buffer. It is understood that acids or bases can be used to adjust the pH of a pharmaceutical composition as needed. It is envisioned that any buffered pH level can be useful in formulating a Clostridial toxin pharmaceutical composition, with the proviso that a therapeutically effective amount of the Clostridial toxin active ingredient, which is preferably a botulinum toxin, is recovered using this effective pH level. In an aspect of this embodiment, an effective pH level is at least about pH 5.0, at least about pH 5.5, at least about pH 6.0, at least about pH 6.5, at least about pH 7.0 or at about about pH 7.5. In another aspect of this embodiment, an effective pH level is at most about pH 5.0, at most about pH 5.5, at most about pH 6.0, at most about pH 6.5, at most about pH 7.0 or at most about pH 7.5. In yet another aspect of this embodiment, an effective pH level is about pH 5.0 to about pH 8.0, an effective pH level is about pH 5.0 to about pH 7.0, an effective pH level is about pH 5.0 to about pH 6.0, is about pH 5.5 to about pH 8.0, an effective pH level is about pH 5.5 to about pH 7.0, an effective pH level is about pH 5.5 to about pH 5.0, is about pH 5.5 to about pH 7.5, an effective pH level is about pH 5.5 to about pH 6.5. An effective pH of 5.5 to 6.0 is particularly preferred.

The pharmaceutical compositions disclosed herein can have a pH of between about 5 and 8 when reconstituted or upon injection. In certain embodiments the composition will have a pH below 8, such as, for example, 7.9, or 7.8, or 7.7, or 7.6, or 7.5, or 7.4, or 7.3, or 7.2, or 7.1, or 7.0, or 6.9, or 6.8, or 6.7, or 6.6, or 6.5, or 6.4, or 6.3, or 6.2, or 6.1, or 6.0, or 5.9, or 5.8, or 5.7, or 5.6, or 5.5, or 5.4, or 5.3, or 5.2, or 5.1, or the like. In some embodiments, the pH ranges from 5 to 7. A pH of 5.5 to 6.5 is preferred and a pH of 5.5 to 6.0 is particularly preferred.

It is envisioned that any concentration of a buffer can be useful in formulating a Clostridial toxin pharmaceutical composition, with the proviso that a therapeutically effective amount of the Clostridial toxin active ingredient, which is most preferably a botulinum toxin, is recovered using this effective concentration of buffer, which is preferably histidine. In aspects of this embodiment, an effective concentration of buffer, which is preferably histidine, is at least 0.1 mM, at least 0.2 mM, at least 0.3 mM, at least 0.4 mM, at least 0.5 mM, at least 0.6 mM, at least 0.7 mM, at least 0.8 mM, or at least 0.9 mM. In other aspects of this embodiment, an effective concentration of buffer, which is preferably histidine, is at least 1.0 mM, at least 2.0 mM, at least 3.0 mM, at least 4.0 mM, at least 5.0 mM, at least 6.0 mM, at least 7.0 mM, at least 8.0 mM, or at least 9.0 mM. In yet other aspects of this embodiment, an effective concentration of buffer, which is preferably histidine, is at least 10 mM, at least 20 mM, at least 30 mM, at least 40 mM, at least 50 mM, at least 60 mM, at least 70 mM, at least 80 mM, or at least 90 mM. In still other aspects of this embodiment, an effective concentration of buffer, which is preferably histidine, is at least 100 mM, at least 200 mM, at least 300 mM, at least 400 mM, at least 500 mM, at least 600 mM, at least 700 mM, at least 800 mM, or at least 900 mM. In further aspects of this embodiment, an effective concentration of buffer, which is preferably histidine, is at most 0.1 mM, at most 0.2 mM, at most 0.3 mM, at most 0.4 mM, at most 0.5 mM, at most 0.6 mM, at most 0.7 mM, at most 0.8 mM, or at most 0.9 mM. In still other aspects of this embodiment, an effective concentration of buffer, which is preferably histidine, is at most 1.0 mM, at most 2.0 mM, at most 3.0 mM, at most 4.0 mM, at most 5.0 mM, at most 6.0 mM, at most 7.0 mM, at most 8.0 mM, or at most 9.0 mM. In yet other aspects of this embodiment, an effective concentration of buffer, which is preferably histidine, is at most 10 mM, at most 20 mM, at most 30 mM, at most 40 mM, at most 50 mM, at most 60 mM, at most 70 mM, at most 80 mM, or at most 90 mM. In still other aspects of this embodiment, an effective concentration of buffer, which is preferably histidine, is at most 100 mM, at most 200 mM, at most 300 mM, at most 400 mM, at most 500 mM, at most 600 mM, at most 700 mM, at most 800 mM, or at most 900 mM. In still further aspects of this embodiment, an effective concentration of buffer, which is preferably histidine, is about 0.1 mM to about 900 mM, 0.1 mM to about 500 mM, 0.1 mM to about 100 mM, 0.1 mM to about 90 mM, 0.1 mM to about 50 mM, 1.0 mM to about 900 mM, 1.0 mM to about 500 mM, 1.0 mM to about 100 mM, 1.0 mM to about 90 mM, or 1.0 mM to about 50 mM. The concentration of the buffer, which is most preferably histidine, is preferably 20 mM.

Embodiments of the invention can be practiced with a composition that comprises a plurality of botulinum toxin serotypes, such as botulinum toxin serotypes selected from the group consisting of botulinum toxin serotypes A, B, C₁ D, E, F, G and mosaic. In certain embodiments, purified botulinum toxins, can be used. In other embodiments, modified botulinum toxins may be used.

In some embodiments, the Clostridial toxin pharmaceutical composition of the invention can be formulated as a lyophilized (i.e. freeze dried) or vacuum dried powder which can be reconstituted with a suitable fluid, such as saline or water, prior to administration to a patient. In alternative embodiments of the invention, the pharmaceutical composition can be formulated as an aqueous solution or suspension.

In some embodiments, the solid Clostridial toxin pharmaceutical composition of the invention comprises a botulinum toxin, a tonicity agent, a poloxamer and/or a polysorbate and an antioxidant. In some embodiments, the Clostridial toxin pharmaceutical composition comprises a botulinum toxin. In some embodiments, the Clostridial toxin pharmaceutical composition comprises trehalose. In some embodiments, the Clostridial toxin pharmaceutical composition comprises poloxamer 188 or polysorbate 20. In some embodiments, the composition comprises EDTA, EGTA, DTPA, or analogs thereof. In alternative embodiments, the composition comprises methionine and/or NAC. In aspects of these alternative embodiments, the composition further comprises EDTA, EGTA, DTPA, or analogs thereof. In some embodiments, the composition further comprises a buffering agent. In one embodiment, the compositon comprises histidine buffer. In some embodiments, the relative weight amounts of trehalose, poloxamer and methionine are within the following ranges respectively: 1 to 10%; 0.5 to 5% and 0.1 to 0.3%. In some embodiments, the relative weight amounts of trehalose, polysorbate and methionine are within the following ranges respectively: 1 to 10%; 0.02% to 0.06%; and 0.1 to 0.3%. In some embodiments, the relative weight amount of EDTA or an EDTA analog is from about 0.01 to 0.10%. In some embodiments, the relative weight amount of NAC ranges from 0.01 to 0.5%.

In aspects of these embodiments, the Clostridial toxin pharmaceutical composition is formulated as a solid (i.e lyophilized or vacuum dried) composition. In some embodiments, the solid Clostridial pharmaceutical composition comprises a lyoprotector. In some embodiments, the preferred lyoprotector includes sucrose, trehalose, mannitol, sorbitol or combinations thereof. In some embodiments, the solid pharmaceutical composition comprises NAC in a relative weight amount of 0.01 to 0.05%. In some embodiments, the pharmaceutical composition further comprises EDTA, EGTA, DTPA, or analogs thereof. In alternative embodiments, the solid pharmaceutical composition comprises methionine and EDTA or an EDTA analog.

In an alternative aspect of these embodiments, the Clostridial toxin pharmaceutical composition is formulated as a liquid. In some embodiments, the liquid pharmaceutical composition comprises NAC in a relative weight amount of 0.1 to 0.5%. In some embodiments the liquid pharmaceucial composition comprises NAC and EDTA, EGTA, DTPA, or analogs thereof. In some embodiments, the liquid pharmaceutical composition comprises histidine buffer. In some embodiments, the liquid pharmaceutical composition has a pH from 5 to 7.

The invention relates to a pharmaceutical composition comprising:
(i) a botulinum toxin;
(ii) poloxamer 188; and
(iii) methionine or N-Acetyl-cysteine.

The combination of poloxamer 188 with either methionine or N-Acetyl-cysteine in the present invention is preferred because of the synergistic effect on the stability of the compositions caused by the combination of this particular surfactant with either of these antioxidants.

The composition may further comprise trehalose or sucrose. Trehalose is preferred over sucrose for the solid compositions.

In addition, the composition may optionally also include NaCl. NaCl may particularly preferably be included in compositions comprising botulinum toxin, trehalose or sucrose, poloxamer 188, and methionine, and is particularly preferably included in liquid compositions comprising botulinum toxin, trehalose or sucrose, poloxamer 188, and methionine. In some lyophilized formulations, NaCl may function as a tonicity agent in a reconstitution vehicle.

In addition to or instead of NaCl, the composition may optionally include EDTA, EGTA, DTPA, or analogs thereof. EDTA may be included in compositions comprising botulinum toxin, trehalose or sucrose, poloxamer 188 and methionine, and is preferably included in liquid compositions comprising botulinum toxin, trehalose or sucrose, poloxamer 188 and methionine.

EDTA, EGTA, DTPA, or analogs thereof may also be included in compositions comprising botulinum toxin, trehalose or sucrose, poloxamer 188 and NAC; and is particularly preferably included in liquid compositions comprising botulinum toxin, trehalose or sucrose, poloxamer 188 and NAC. The combination of EDTA and NAC is particularly preferred as it has a synergistic stabilising effect on the compositions of the invention.

The relative weight amounts (%, w/w) of trehalose or sucrose, poloxamer 188, methionine, and NaCl where present, and EDTA where present are preferably within the following ranges:

| | |
|---|---|
| Trehalose or sucrose | 1 to 10 |
| poloxamer 188 | 0.5 to 5 |
| methionine | 0.1 to 0.3 |
| NaCl | 0.1 to 10 |
| EDTA | 0.01 to 0.1. |

Such compositions may be both liquid and solid formulations. When the composition is a solid composition, it is preferred that the sugar is trehalose.

In addition, these compositions preferably comprise a buffer. In compositions of the invention, the buffer is preferably histidine, and the concentration of histidine is preferably 20 mM when the composition is a liquid composition. In these compositions, the pH is preferably in the range 5-7, the pH is more preferably in the range 5.5-6.5, and the pH is most preferably in the range 5.5-6. Meanwhile, when the composition is a solid, the histidine is preferably comprised in a relative weight amount of 0.1 to 0.5 (% w/w), more preferably in a relative weight amount of 0.3-0.4% (w/w).

More preferably, the relative weight amounts (%, w/w) of trehalose, poloxamer 188, and methionine are:
a)

| | |
|---|---|
| trehalose | 7 to 9 |
| poloxamer 188 | 3.5 to 4.5 |
| methionine | 0.15 to 0.25; |

and are preferably

| | |
|---|---|
| trehalose | 8 |
| poloxamer 188 | 4 |
| methionine | 0.2. |

In the invention, the composition labelled as a) above may be a solid composition, which is preferably lyophilised. This composition also preferably comprises a buffer, wherein the buffer is preferably histidine, wherein the histidine is preferably comprised in the solid composition in a relative weight amount of 0.1 to 0.5 (% w/w), more preferably in a relative weight amount of 0.3-0.4% (w/w). The histidine may be present as a pure substance, the acid salt of histidine (e.g. Histidine HCl), a hydrate of an acid salt of the histidine, or a mix of any of the foregoing. In a preferred embodiment, histidine base may be present in an amount of 1.43 mg/mL (or 0.14% w/w) together with histidine hydrochloride in an amount of 2.26 mg/mL (or 0.23% w/w). The total concentration of histidine species (combined histidine concentration) is then 0.37% w/w. In one embodiment, the solid composition is reconstituted with a reconstitution vehicle comprising NaCl prior to administration to a patient.

This solid composition most preferably has either of the following compositions:

**Table A**

| Raw Material (Ingredient) | Concentration, w/w | Quantity per vial | Function |
|---|---|---|---|
| Botulinum toxin type A | 1200 U/mL | 200U | Drug Substance |
| Trehalose | 80 mg/mL | 16 mg | Lyo Protectant |
| P188 | 40 mg/mL | 8 mg | Surfactant |
| L-methionine | 2 mg/mL | 0.4 mg | Antioxidant |
| L-Histidine | 1.430 mg/mL | 0.286 mg | Buffer |
| L-Histidine HCl, Monohydrate | 2.263 mg/mL | 0.453 mg | Buffer |
| Water | QS Adjust | QS Adjust | Solvent, removed by freeze-drying |

In one embodiment of the invention, the composition is a solid composition consisting of one or more botulinum toxins, trehalose, poloxamer 188, methionine, and a buffer, wherein these components are preferably present in the amounts defined above. In one embodiment, the solid composition is reconstituted with a reconstitution vehicle comprising NaCl prior to administration to a patient. In one embodiment, NaCl may be present in an amount of 0.9% (w/w) in the reconstitution vehicle.

In the invention, the composition labelled as a) above may also be a liquid composition. In this case, the composition preferably comprises a buffer, wherein the buffer is preferably histidine. The concentration of histidine is preferably 20 mM, and the pH is preferably in the range 5-7, more preferably in the range 5.5-6.5, and the pH is yet more preferably in the range 5.5-6, and is most preferably 6.

In one embodiment of the invention, the composition is a liquid composition consisting of one or more botulinum toxins, trehalose, poloxamer 188, methionine, and a buffer, wherein these components are preferably present in the amounts defined above.

Also more preferably, the relative weight amounts (%, w/w) of trehalose, poloxamer 188 and methionine are:
b)

| | |
|---|---|
| trehalose | 7 to 9 |
| poloxamer 188 | 0.5 to 0.7 |
| methionine | 0.15 to 0.25; |

and are preferably

| | |
|---|---|
| trehalose | 8 |
| poloxamer 188 | 0.6 |
| methionine | 0.2. |

In the invention, the composition labelled as b) above is preferably a liquid composition. In this case, the composition preferably comprises a buffer, wherein the buffer is preferably histidine. The concentration of histidine is preferably 20 mM, and the pH is preferably in the range 5-7, the pH is more preferably in the range 5.5-6.5, and the pH is yet more preferably in the range 5.5-6, and is most preferably 5.5.

In one embodiment of the invention, the composition is a liquid composition consisting of one or more botulinum toxins, trehalose, poloxamer 188, methionine, and a buffer, wherein these components are preferably present in the amounts defined above.

Also more preferably, the relative weight amounts (%, w/w) of trehalose, poloxamer 188, methionine, and NaCl are:
c)

| | |
|---|---|
| trehalose | 1 to 3 |
| poloxamer 188 | 3.5 to 4.5 |
| methionine | 0.15 to 0.25 |
| NaCl | 0.4 to 0.8; |

and are preferably

| | |
|---|---|
| trehalose | 2 |
| poloxamer 188 | 4 |
| methionine | 0.2 |
| NaCl | 0.6. |

In the invention, the composition labelled as c) above is preferably a liquid composition. In this case, the composition preferably comprises a buffer, wherein the buffer is preferably histidine. The concentration of histidine is preferably 20 mM, and the pH is preferably in the range 5-7, and the pH is more preferably in the range 5.5-6.5, the pH is yet more preferably in the range 5.5-6, and is most preferably 5.5.

In one embodiment of the invention, the composition is a liquid composition consisting of one or more botulinum toxins, trehalose, poloxamer 188, methionine, NaCl and a buffer, wherein these components are preferably present in the amounts defined above.

Also more preferably, the relative weight amounts (%, w/w) of trehalose, poloxamer 188, methionine, and EDTA are:
d)

| | |
|---|---|
| trehalose | 7 to 9 |
| poloxamer 188 | 3.5 to 4.5 |
| methionine | 0.15 to 0.25 |
| EDTA | 0.01 to 0.05; |

and are preferably

| | |
|---|---|
| trehalose | 8 |
| poloxamer 188 | 4 |
| methionine | 0.2 |
| EDTA | 0.03. |

In the invention, the composition labelled as d) above is preferably a liquid composition. In this case, the composition preferably comprises a buffer, wherein the buffer is preferably histidine. The concentration of histidine is preferably 20 mM, and the pH is preferably in the range 5-7, the pH is more preferably in the range 5.5-6.5, and the pH is yet more preferably in the range 5.5-6, and is most preferably 6.

In one embodiment of the invention, the composition is a liquid composition consisting of one or more botulinum toxins, trehalose, poloxamer 188, methionine, EDTA and a buffer, wherein these components are preferably present in the amounts defined above.

The relative weight amounts (%, w/w) of trehalose or sucrose, poloxamer 188, N-Acetyl-cysteine, and EDTA where present are preferably within the following ranges

| | |
|---|---|
| trehalose or sucrose | 1 to 10 |
| poloxamer 188 | 0.5 to 5 |
| N-Acetyl-cysteine | 0.01 to 0.5, |
| EDTA | 0.01 to 0.1. |

and
Such compositions may be both liquid and solid formulations, and are preferably liquid compositions. When the composition is a solid composition, it is preferred that the sugar is trehalose.

In addition, these compositions preferably comprise a buffer. In compositions of the invention, the buffer is preferably histidine, and the concentration of histidine is preferably 20 mM when the composition is a liquid composition. In these compositions, the pH is preferably in the range 5-7, the pH is more preferably in the range 5.5-6.5, and the pH is most preferably in the range 5.5-6. Meanwhile, when the composition is a solid, the histidine is preferably comprised in a relative weight amount of 0.1 to 0.5 (% w/w), more preferably in a relative weight amount of 0.3-0.4% (w/w).

More preferably, the relative weight amounts (%, w/w) of trehalose, poloxamer 188, N-Acetyl-cysteine and EDTA are:

| | |
|---|---|
| trehalose | 7 to 9 |
| poloxamer 188 | 3.5 to 4.5 |
| N-Acetyl-cysteine | 0.1 to 0.3 and |
| EDTA | 0.01 to 0.05; |

and are preferably

| | |
|---|---|
| trehalose | 8 |
| poloxamer 188 | 4 |
| N-Acetyl-cysteine | 0.2 and |
| EDTA | 0.03. |

In the invention, this composition is preferably a liquid composition. In this case, the composition preferably comprises a buffer, wherein the buffer is preferably histidine. The concentration of histidine is preferably 20 mM, and the pH is preferably in the range 5-7, the pH is more preferably in the range 5.5-6.5, and the pH is yet more preferably in the range 5.5-6, and is most preferably 6.

In one embodiment of the invention, the composition is a liquid composition consisting of one or more botulinum toxins, trehalose, poloxamer 188, N-Acetyl-cysteine, EDTA and a buffer, wherein these components are preferably present in the amounts defined above.

As described above, the EDTA is an optional component in the N-Acetyl-cysteine containing compositions of the invention. In such an EDTA-free composition, the relative weight amounts (%, w/w) of trehalose, poloxamer 188, and N-Acetyl-cysteine may be:

| | |
|---|---|
| trehalose | 7 to 9 |
| poloxamer 188 | 3.5 to 4.5 |
| N-Acetyl-cysteine | 0.1 to 0.3; |

and are preferably

| | |
|---|---|
| trehalose | 8 |
| poloxamer 188 | 4 |
| N-Acetyl-cysteine | 0.2. |

Alternatively, the relative weight amounts (%, w/w) of components in the EDTA-free compositions are:

| | |
|---|---|
| trehalose | 1 to 3 |
| poloxamer 188 | 3.5 to 4.5 |
| N-Acetyl-cysteine | 0.01 to 0.05; |

and are preferably:

| | |
|---|---|
| trehalose | 2 |
| poloxamer 188 | 4 |
| N-Acetyl-cysteine | 0.03. |

In the invention, this EDTA-free composition is preferably a solid composition, which is preferably lyophilised. This composition also preferably comprises a buffer, wherein the buffer is preferably histidine, wherein the histidine is preferably comprised in the solid composition in a relative weight amount of 0.1 to 0.5 (% w/w), more preferably in a relative weight amount of 0.3-0.4% (w/w). The histidine may be present as a pure substance, the acid salt of histidine, a hydrate of an acid salt of the histidine, or a mix of any of the foregoing. In a preferred embodiment, histidine base may be present in an amount of 1.43 mg/mL (or 0.14% w/w) together with histidine hydrochloride in an amount of 2.26 mg/mL (or 0.23% w/w). The total concentration of histidine species (combined histidine concentration) is then 0.37% w/w.

In one embodiment of the invention, the composition is a solid composition consisting of one or more botulinum toxins, trehalose, poloxamer 188, N-Acetyl-cysteine, and a buffer, wherein these components are preferably present in the amounts defined above.

In some cases, the composition of the invention does not comprise a sugar or polyalcohol. As a result, the composition comprises botulinum toxin, poloxamer 188, and methionine or N-Acetyl-cysteine, but no sugar or polyalcohol. When the composition comprises N-Acetyl-cysteine, it is preferred that it additionally also contains EDTA.

In such compositions, the relative weight amounts (%, w/w) of poloxamer 188, and methionine are preferably within the following ranges:

| | |
|---|---|
| poloxamer 188 | 0.5 to 5 |
| methionine | 0.1 to 0.3. |

Meanwhile, the relative weight amounts (%, w/w) of poloxamer 188, and N-Acetyl-cysteine and EDTA are preferably within the following ranges:

| | |
|---|---|
| poloxamer 188 | 0.5 to 5 |
| N-Acetyl-cysteine | 0.01 to 0.5 |
| EDTA | 0.01 to 0.1. |

Such compositions are preferably liquid formulations.

More preferably, the relative weight amounts (%, w/w) of poloxamer 188 and methionine are:

| | |
|---|---|
| poloxamer 188 | 3.5 to 4.5 |
| methionine | 0.15 to 0.25; |

and are preferably

| | |
|---|---|
| poloxamer 188 | 4 |
| methionine | 0.2. |

As explained above, such compositions are preferably liquid compositions. In this case, the composition preferably comprises a buffer, wherein the buffer is preferably histidine. The concentration of histidine is preferably 20 mM, and the pH is preferably in the range 5-7, the pH is more preferably in the range 5.5-6.5, and the pH is yet more preferably in the range 5.5-6, and is most preferably 6. These compositions may also include NaCl. NaCl may be included in a relative weight amount of 0.5 to 1.5% (w/w), more preferably 0.9% (w/w).

In one embodiment of the invention, the composition is a liquid composition consisting of one or more botulinum toxins, poloxamer 188, methionine, and a buffer, and optionally NaCl, wherein these components are preferably present in the amounts defined above.

It is also preferred that the relative weight amounts (%, w/w) of poloxamer 188 and N-Acetyl-cysteine are:

| | |
|---|---|
| poloxamer 188 | 3.5 to 4.5 |
| N-Acetyl-cysteine | 0.1 to 0.3, and optionally |
| EDTA | 0.01 to 0.05; |

and are preferably

| | |
|---|---|
| poloxamer 188 | 4 |
| N-Acetyl-cysteine | 0.2, and optionally |
| EDTA | 0.03. |

Such compositions are preferably liquid compositions. In this case, the composition preferably comprises a buffer, wherein the buffer is preferably histidine. The concentration of histidine is preferably 20 mM, and the pH is preferably in the range 5-7, the pH is more preferably in the range 5.5-6.5, and the pH is yet more preferably in the range 5.5-6, and is most preferably 6. These compositions may also include NaCl. NaCl may be included in a relative weight amount of 0.5 to 1.5% (w/w), more preferably 0.9% (w/w).

In one embodiment of the invention, the composition is a liquid composition consisting of one or more botulinum toxins, poloxamer 188, N-Acetyl-cysteine, a buffer, and optionally EDTA, and optionally NaCl, wherein these components are preferably present in the amounts defined above.

In another embodiment, the composition which does not comprise a sugar or polyalcohol described above may comprise Tween-20 in the place of poloxamer-188. Such compositions may comprise Tween-20 in an amount of 0.01 to 0.1% (w/w), more preferably 0.02 to 0.06% (w/w), and most preferably 0.04% (w/w).

In another preferred aspect of the invention, the relative weight amounts (%, w/w) of sucrose, poloxamer 188, and methionine are:
a')

| | |
|---|---|
| sucrose | 7 to 9 |
| poloxamer 188 | 3.5 to 4.5 |
| methionine | 0.15 to 0.25; |

and are preferably

| | |
|---|---|
| sucrose | 8 |
| poloxamer 188 | 4 |
| methionine | 0.2. |

In the invention, the composition labelled as a') above may be a solid composition, which is preferably lyophilised. This composition also preferably comprises a buffer, wherein the buffer is preferably histidine, wherein the histidine is preferably comprised in the solid composition in a relative weight amount of 0.1 to 0.5 (% w/w), more preferably in a relative weight amount of 0.3-0.4% (w/w). The histidine may be present as a pure substance, the acid salt of histidine (e.g. Histidine HCl), a hydrate of an acid salt of the histidine, or a mix of any of the foregoing. In a preferred embodiment, histidine base may be present in an amount of 1.43 mg/mL (or 0.14% w/w) together with histidine hydrochloride in an amount of 2.26 mg/mL (or 0.23% w/w). The total concentration of histidine species (combined histidine concentration) is then 0.37% w/w.

This solid composition most preferably has either of the following compositions:

**Table B**

| Raw Material (Ingredient) | Concentration, w/w | Quantity per vial | Function |
|---|---|---|---|
| Botulinum toxin type A | 1200 U/mL | 200U | Drug Substance |
| Sucrose | 80 mg/mL | 16 mg | Lyo Protectant |
| P188 | 40 mg/mL | 8 mg | Surfactant |
| L-methionine | 2 mg/mL | 0.4 mg | Antioxidant |
| L-Histidine | 1.430 mg/mL | 0.286 mg | Buffer |
| L-Histidine HCl, Monohydrate | 2.263 mg/mL | 0.453 mg | Buffer |
| Water | QS Adjust | QS Adjust | Solvent, removed by freeze-drying |

In one embodiment of the invention, the composition is a solid composition consisting of one or more botulinum toxins, sucrose, poloxamer 188, methionine, and a buffer, wherein these components are preferably present in the amounts defined above. In one embodiment, the solid composition is reconstituted with a reconstitution vehicle comprising NaCl prior to administration to a patient. In one embodiment, NaCl may be present in an amount of 0.9% (w/w) in the reconstitution vehicle.

In the invention, the composition labelled as a') above may also be a liquid composition. In this case, the composition preferably comprises a buffer, wherein the buffer is preferably histidine. The concentration of histidine is preferably 20 mM, and the pH is preferably in the range 5-7, the pH is more preferably in the range 5.5-6.5, and the pH is yet more preferably in the range 5.5-6, and is most preferably 6.

In one embodiment of the invention, the composition is a liquid composition consisting of one or more botulinum toxins, sucrose, poloxamer 188, methionine, and a buffer, wherein these components are preferably present in the amounts defined above.

Also more preferably, the relative weight amounts (%, w/w) of sucrose, poloxamer 188 and methionine are:
b')

| | |
|---|---|
| sucrose | 7 to 9 |
| poloxamer 188 | 0.5 to 0.7 |
| methionine | 0.15 to 0.25; |

and are preferably

| | |
|---|---|
| sucrose | 8 |
| poloxamer 188 | 0.6 |
| methionine | 0.2. |

In the invention, the composition labelled as b') above is preferably a liquid composition. In this case, the composition preferably comprises a buffer, wherein the buffer is preferably histidine. The concentration of histidine is preferably 20 mM, and the pH is preferably in the range 5-7, the pH is more preferably in the range 5.5-6.5, and the pH is yet more preferably in the range 5.5-6, and is most preferably 5.5.

In one embodiment of the invention, the composition is a liquid composition consisting of one or more botulinum toxins, sucrose, poloxamer 188, methionine, and a buffer, wherein these components are preferably present in the amounts defined above.

Also more preferably, the relative weight amounts (%, w/w) of sucrose, poloxamer 188, methionine, and NaCl are:
c')

| | |
|---|---|
| sucrose | 1 to 3 |
| poloxamer 188 | 3.5 to 4.5 |
| methionine | 0.15 to 0.25 |
| NaCl | 0.4 to 0.8; |

and are preferably

| | |
|---|---|
| sucrose | 2 |
| poloxamer 188 | 4 |
| methionine | 0.2 |
| NaCl | 0.6. |

In the invention, the composition labelled as c') above is preferably a liquid composition. In this case, the composition preferably comprises a buffer, wherein the buffer is preferably histidine. The concentration of histidine is preferably 20 mM, and the pH is preferably in the range 5-7, the pH is more preferably in the range 5.5-6.5, and the pH is yet more preferably in the range 5.5-6, and is most preferably 5.5.

In one embodiment of the invention, the composition is a liquid composition consisting of one or more botulinum toxins, sucrose, poloxamer 188, methionine, NaCl and a buffer, wherein these components are preferably present in the amounts defined above.

Also more preferably, the relative weight amounts (%, w/w) of sucrose, poloxamer 188, methionine, and EDTA are:
d')

| | |
|---|---|
| sucrose | 7 to 9 |
| poloxamer 188 | 3.5 to 4.5 |
| methionine | 0.15 to 0.25 |
| EDTA | 0.01 to 0.05; |

and are preferably

| | |
|---|---|
| sucrose | 8 |
| poloxamer 188 | 4 |
| methionine | 0.2 |
| EDTA | 0.03. |

In the invention, the composition labelled as d') above is preferably a liquid composition. In this case, the composition preferably comprises a buffer, wherein the buffer is preferably histidine. The concentration of histidine is preferably 20 mM, and the pH is preferably in the range 5-7, the pH is more preferably in the range 5.5-6.5, and the pH is more preferably in the range 5.5-6, and is most preferably 6.

In one embodiment of the invention, the composition is a liquid composition consisting of one or more botulinum toxins, sucrose, poloxamer 188, methionine, EDTA and a buffer, wherein these components are preferably present in the amounts defined above.

In another preferred embodiment, the relative weight amounts (%, w/w) of sucrose, poloxamer 188, N-Acetyl-cysteine and EDTA are:

| | |
|---|---|
| sucrose | 7 to 9 |
| poloxamer 188 | 3.5 to 4.5 |
| N-Acetyl-cysteine | 0.1 to 0.3 and |
| EDTA | 0.01 to 0.05; |

and are preferably

| | |
|---|---|
| sucrose | 8 |
| poloxamer 188 | 4 |
| N-Acetyl-cysteine | 0.2 and |
| EDTA | 0.03. |

In the invention, this composition is preferably a liquid composition. In this case, the composition preferably comprises a buffer, wherein the buffer is preferably histidine. The concentration of histidine is preferably 20 mM, and the pH is preferably in the range 5-7, the pH is more preferably in the range 5.5-6.5, and the pH is yet more preferably in the range 5.5-6, and is most preferably 6.

In one embodiment of the invention, the composition is a liquid composition consisting of one or more botulinum toxins, sucrose, poloxamer 188, N-Acetyl-cysteine, EDTA and a buffer, wherein these components are preferably present in the amounts defined above.

As described above, the EDTA is an optional component in the N-Acetyl-cysteine containing compositions of the invention. In such an EDTA-free composition, the relative weight amounts (%, w/w) of sucrose, poloxamer 188, and N-Acetyl-cysteine may be:

| | |
|---|---|
| sucrose | 7 to 9 |
| poloxamer 188 | 3.5 to 4.5 |
| N-Acetyl-cysteine | 0.1 to 0.3; |

and are preferably

| | |
|---|---|
| sucrose | 8 |
| poloxamer 188 | 4 |
| N-Acetyl-cysteine | 0.2. |

Alternatively, the relative weight amounts (%, w/w) of components in the EDTA-free compositions are:

| | |
|---|---|
| sucrose | 1 to 3 |
| poloxamer 188 | 3.5 to 4.5 |
| N-Acetyl-cysteine | 0.01 to 0.05; |

and are preferably:

| | |
|---|---|
| sucrose | 2 |
| poloxamer 188 | 4 |
| N-Acetyl-cysteine | 0.03. |

In the invention, this EDTA-free composition is preferably a solid composition, which is preferably lyophilised. This composition also preferably comprises a buffer, wherein the buffer is preferably histidine, wherein the histidine is preferably comprised in the solid composition in a relative weight amount of 0.1 to 0.5 (% w/w), more preferably in a relative weight amount of 0.3-0.4% (w/w). The histidine may be present as a pure substance, the acid salt of histidine, a hydrate of an acid salt of the histidine, or a mix of any of the foregoing. In a preferred embodiment, histidine base may be present in an amount of 1.43 mg/mL (or 0.14% w/w) together with histidine hydrochloride in an amount of 2.26 mg/mL (or 0.23% w/w). The total concentration of histidine species (combined histidine concentration) is then 0.37% w/w.

In one embodiment of the invention, the composition is a solid composition consisting of one or more botulinum toxins, sucrose, poloxamer 188, N-Acetyl-cysteine, and a buffer, wherein these components are preferably present in the amounts defined above.

The compositions of the invention may be a liquid composition, in which case the composition preferably comprises a buffer. This buffer is preferably histidine, and the concentration of histidine is preferably 20 mM. The pH is preferably in the range 5-7, the pH is more preferably in the range 5.5-6.5, and is most preferably in the range 5.5-6.

The compositions of the invention may also be a solid composition, which is preferably lyophilised. The composition may further comprise a buffer, wherein the buffer is preferably histidine, wherein the histidine is preferably comprised in a relative weight amount of 0.1 to 0.5 (% w/w), more preferably in a relative weight amount of 0.3-0.4% (w/w).

It is particularly preferred that the composition of the invention comprises no animal-derived protein.

Any of the foregoing compositions comprising a botulinum toxin, poloxamer 188, and methionine or N-Acetyl-cysteine may also further comprise ethylene diamine tetraacetic acid sodium salt (EDTA) or an EDTA analog. The relative weight amount (%, w/w) of EDTA in these compositions may be in the range from about 0.01 to 0.10.

In the present invention, the term "Antioxidant" refers to any compound which protects an active ingredient from reaction with oxygen. Antioxidants can be broadly divided into three categories:
(i) sacrificial antioxidants, which react with oxygen more readily than a particular active ingredient and therefore can scavenge oxygen. In the invention, this type of antioxidant is preferably ascorbic acid;
(ii) chain terminators, which react with free radicals and peroxides to prevent propagation of radical chains. In the invention, methionine, cysteine, N-Acetyl-cysteine and BHT are preferable chain terminators; and
(iii) chelating agents, which reduce catalytic activity of transition metals by forming complexes with the metals. In the invention, EDTA, EGTA, and DTPA are preferred chelating agents.

The following compositions define the invention by reference to the general or specific types of antioxidant discussed in the previous paragraph. These compositions may all optionally comprise a buffer. In the following liquid compositions, the buffer is preferably histidine, and the concentration of histidine is preferably 20 mM, and the pH is preferably in the range 5-7, and the pH is still more preferably in the range 5.5-6.5, and the pH is most preferably in the range 5.5-6. In the following solid compositions, the buffer is preferably histidine, and the histidine is preferably comprised in a relative weight amount of 0.1 to 0.5 (% w/w), and is more preferably contained in a relative weight amount of 0.3-0.4% (w/w). In addition, all of the following compositions may comprise NaCl.

The invention also relates to a liquid pharmaceutical composition, comprising:
(i) a Clostridial toxin;
(ii) a poloxamer;
(iii) a chelating agent; and
(iv) a sacrificial antioxidant.

In a preferred embodiment of this liquid pharmaceutical composition, the chelating agent is EDTA, EGTA or DTPA and the sacrificial antioxidant is ascorbic acid. In such a composition, the clostridial toxin is preferably a botulinum toxin and the poloxamer is preferably poloxamer-188.

The invention also relates to a liquid pharmaceutical composition, comprising:
(i) a Clostridial toxin;
(ii) a poloxamer;
(iii) a chelating agent; and/or
(iv) a chain terminator.

In a preferred embodiment of this liquid pharmaceutical composition, the chelating agent is EDTA and the chain terminator is N-acetyl-cysteine. In another preferred embodiment, the chelating agent is EDTA and the chain terminator is Butylated hydroxytoluene (BHT). In another preferred embodiment, the chelating agent is EDTA and the chain terminator is methionine. In another preferred embodiment of this liquid pharmaceutical composition, the chelating agent is DTPA and the chain terminator is N-acetyl-cysteine. In another preferred embodiment, the chelating agent is DTPA and the chain terminator is BHT. In another preferred embodiment, the chelating agent is DTPA and the chain terminator is methionine. In another preferred embodiment of this liquid pharmaceutical composition, the chelating agent is EGTA and the chain terminator is N-acetyl-cysteine. In another preferred embodiment, the chelating agent is EGTA and the chain terminator is BHT. In another preferred embodiment, the chelating agent is EGTA and the chain terminator is methionine. In another preferred embodiment, the liquid composition includes a chain terminator and not a chelating agent; wherein the chain terminator is NAC. In all of these liquid compositions, the clostridial toxin is preferably a botulinum toxin and the poloxamer is preferably poloxamer-188.

The invention also relates to a liquid pharmaceutical composition comprising:
(i) a Clostridial toxin;
(ii) a poloxamer; and
(iii) methionine.

In this embodiment, the clostridial toxin is preferably a botulinum toxin and the poloxamer is preferably poloxamer-188. In such compositions, the poloxamer-188 and methionine are preferably present in the amounts defined above with respect to the compositions which do not comprise a sugar or a polyalcohol.

The invention also relates to a liquid pharmaceutical composition comprising:
(i) a Clostridial toxin;
(ii) a tonicity agent selected from trehalose, sucrose, sodium chloride, mannitol, sorbitol, glucose, and combinations thereof;
(ii) a poloxamer; and
(iii) methionine.

In this embodiment, the clostridial toxin is preferably a botulinum toxin and the poloxamer is preferably poloxamer-188. In such compositions, the poloxamer-188 and methionine are preferably present in the amounts defined above with respect to the compositions which comprise a sugar or a polyalcohol.

The invention also preferably relates to a liquid pharmaceutical composition, comprising:
(i) a Clostridial toxin;
(ii) a tonicity agent selected from trehalose, sucrose, sodium chloride, mannitol, sorbitol, glucose, and combinations thereof;
(iii) a surfactant selected from a poloxamer, a polysorbate and combinations thereof;
(iv) a chelating agent; and
(v) a sacrificial antioxidant.

In this particular embodiment, the chelating agent is preferably EDTA, EGTA or DTPA and the sacrificial antioxidant is preferably ascorbic acid. The clostridial toxin is preferably a botulinum toxin, the poloxamer is preferably poloxamer-188 and the polysorbate is preferably polysorbate-20 or 80.

The invention also preferably relates to a liquid pharmaceutical composition, comprising:
(i) a Clostridial toxin;
(ii) a tonicity agent selected from trehalose, sucrose, sodium chloride, mannitol, sorbitol, glucose, and combinations thereof;
(iii) a surfactant selected from a poloxamer, a polysorbate and combinations thereof;
(iv) a chelating agent; and
(v) a chain terminator.

In this particular embodiment, the chelating agent is preferably EDTA, EGTA or DTPA and the chain terminator is preferably NAC. The clostridial toxin is preferably a botulinum toxin, the poloxamer is preferably poloxamer-188 and the polysorbate is preferably polysorbate-20 or 80.

The invention also preferably relates to a liquid pharmaceutical composition, comprising:
(i) a Clostridial toxin;
(ii) a tonicity agent selected from trehalose, sucrose, NaCl and combinations thereof;
(iii) a surfactant selected from a poloxamer, a polysorbate and combinations thereof;
(iv) a chelating agent; and
(v) a chain terminator.

In this particular embodiment, the chelating agent is preferably EDTA, EGTA or DTPA and the chain terminator is preferably NAC. The clostridial toxin is preferably a botulinum toxin, the poloxamer is preferably poloxamer-188 and the polysorbate is preferably polysorbate-20 or 80.

The invention also relates to a lyophilized pharmaceutical composition, comprising:
(i) a Clostridial toxin;
(ii) a lyoprotectant selected from trehalose, sucrose, mannitol, sorbitol, glucose, and combinations thereof;
(iii) a surfactant selected from a poloxamer, a polysorbate and combinations thereof;
(iv) a chelating agent; and
(v) a sacrificial antioxidant.

In this particular embodiment, the chelating agent is preferably EDTA, EGTA or DTPA and the sacrificial antioxidant is preferably ascorbic acid. The clostridial toxin is preferably a botulinum toxin, the poloxamer is preferably poloxamer-188 and the polysorbate is preferably polysorbate-20 or 80. In certain embodiments, the lyophilized composition is reconstituted with a tonicity agent selected from trehalose, sucrose, sodium chloride, mannitol, sorbitol, glucose, and combinations thereof. In at least one embodiment, the lyophilized composition is reconstituted with a reconstitution vehicle comprising NaCl prior to administration to a patient. In at least one embodiment, NaCl is present in an amount of 0.9% (w/w) in the reconstitution vehicle.

The invention also relates to a lyophilized pharmaceutical composition, comprising:
(i) a Clostridial toxin;
(ii) a lyoprotectant selected from trehalose, sucrose, mannitol, sorbitol, glucose, and combinations thereof;
(iii) a surfactant selected from a poloxamer, a polysorbate and combinations thereof;
(iv) a chelating agent; and
(v) a chain terminator.

In this particular embodiment, the chelating agent is preferably EDTA, EGTA or DTPA and the chain terminator is preferably NAC. The clostridial toxin is preferably a botulinum toxin, the poloxamer is preferably poloxamer-188 and the polysorbate is preferably polysorbate-20 or 80. In certain embodiments, the lyophilized composition is reconstituted with a tonicity agent selected from trehalose, sucrose, sodium chloride, mannitol, sorbitol, glucose, and combinations thereof. In at least one embodiment, the lyophilized composition is reconstituted with a reconstitution vehicle comprising NaCl prior to administration to a patient. In at least one embodiment, NaCl is present in an amount of 0.9% (w/w) in the reconstitution vehicle.

The invention also relates to a lyophilized pharmaceutical composition, comprising:
(i) a Clostridial toxin;
(ii) a lyoprotectant selected from trehalose, sucrose, mannitol, sorbitol, glucose, and combinations thereof;
(iii) a surfactant selected from a poloxamer, a polysorbate and combinations thereof; and
(iv) a chain terminator.

In this particular embodiment, the chain terminator is preferably NAC or methionine. The clostridial toxin is preferably a botulinum toxin, the poloxamer is preferably poloxamer-188 and the polysorbate is preferably polysorbate-20 or 80. In certain embodiments, the lyophilized composition is reconstituted with a tonicity agent selected from trehalose, sucrose, sodium chloride, mannitol, sorbitol, glucose, and combinations thereof. In at least one embodiment, the lyophilized composition is reconstituted with a reconstitution vehicle comprising NaCl prior to administration to a patient. In at least one embodiment, NaCl is present in an amount of 0.9% (w/w) in the reconstitution vehicle.

The invention also relates to a lyophilized pharmaceutical composition, comprising:
(i) a Clostridial toxin;
(ii) trehalose, sucrose, sodium chloride, mannitol, sorbitol, glucose, or combinations thereof;
(iii) a surfactant selected from a poloxamer, a polysorbate and combinations thereof;
(iv) a chelating agent; and
(v) a chain terminator.

In this particular embodiment, the chelating agent is preferably EDTA, EGTA or DTPA and the chain terminator is preferably NAC. The clostridial toxin is preferably a botulinum toxin, the poloxamer is preferably poloxamer-188 and the polysorbate is preferably polysorbate-20 or 80. In certain embodiments, the lyophilized composition is reconstituted with a tonicity agent selected from trehalose, sucrose, sodium chloride, mannitol, sorbitol, glucose, and combinations thereof. In at least one embodiment, the lyophilized composition is reconstituted with a reconstitution vehicle comprising NaCl prior to administration to a patient. In at least one embodiment, NaCl is present in an amount of 0.9% (w/w) in the reconstitution vehicle.

The invention also relates to a lyophilized pharmaceutical composition, comprising:
(i) a Clostridial toxin;
(ii) trehalose, sucrose, sodium chloride, mannitol, sorbitol, glucose, or combinations thereof;
(iii) a poloxamer;
(iv) a chelating agent; and
(v) a sacrificial antioxidant.

In this particular embodiment, the chelating agent is preferably EDTA, EGTA or DTPA and the sacrificial antioxidant is preferably ascorbic acid. The clostridial toxin is preferably a botulinum toxin and the poloxamer is preferably poloxamer-188. In certain embodiments, the lyophilized composition is reconstituted with a tonicity agent selected from trehalose, sucrose, sodium chloride, mannitol, sorbitol, glucose, and combinations thereof. In at least one embodiment, the lyophilized composition is reconstituted with a reconstitution vehicle comprising NaCl prior to administration to a patient. In at least one embodiment, NaCl is present in an amount of 0.9% (w/w) in the reconstitution vehicle.

The invention also relates to a lyophilized pharmaceutical composition, comprising:
(i) a Clostridial toxin;
(ii) trehalose, sucrose, sodium chloride, mannitol, sorbitol, glucose, or combinations thereof;
(iii) a poloxamer;
(iv) a chelating agent; and
(v) a chain terminator.

In a preferred embodiment, that chelating agent is EDTA, and the chain terminator is N-Acetyl-cysteine. The clostridial toxin is preferably a botulinum toxin and the poloxamer is preferably poloxamer-188. In certain embodiments, the lyophilized composition is reconstituted with a tonicity agent selected from trehalose, sucrose, sodium chloride, mannitol, sorbitol, glucose, and combinations thereof. In at least one embodiment, the lyophilized composition is reconstituted with a reconstitution vehicle comprising NaCl prior to administration to a patient. In at least one embodiment, NaCl is present in an amount of 0.9% (w/w) in the reconstitution vehicle.

The invention also relates to a lyophilized pharmaceutical composition, comprising:
(i) a Clostridial toxin;
(ii) trehalose, sucrose, sodium chloride, mannitol, sorbitol, glucose, or combinations thereof;
(iii) a poloxamer; and
(iv) a chain terminator.

In a preferred embodiment, the chain terminator is N-Acetyl-cysteine. The clostridial toxin is preferably a botulinum toxin and the poloxamer is preferably poloxamer-188. In certain embodiments, the lyophilized composition is reconstituted with a tonicity agent selected from trehalose, sucrose, sodium chloride, mannitol, sorbitol, glucose, and combinations thereof. In at least one embodiment, the lyophilized composition is reconstituted with a reconstitution vehicle comprising NaCl prior to administration to a patient. In at least one embodiment, NaCl is present in an amount of 0.9% (w/w) in the reconstitution vehicle.

The invention also relates to a lyophilized pharmaceutical composition, comprising:
(i) a Clostridial toxin;
(ii) trehalose, sucrose, sodium chloride, mannitol, sorbitol, glucose, or combinations thereof;
(iii) a poloxamer; and
(iv) methionine.

In a preferred embodiment, the clostridial toxin is preferably a botulinum toxin and the poloxamer is preferably poloxamer-188. In certain embodiments, the lyophilized composition is reconstituted with a tonicity agent selected from trehalose, sucrose, sodium chloride, mannitol, sorbitol, glucose, and combinations thereof. In at least one embodiment, the lyophilized composition is reconstituted with a reconstitution vehicle comprising NaCl prior to administration to a patient. In at least one embodiment, NaCl is present in an amount of 0.9% (w/w) in the reconstitution vehicle.

The invention also relates to a lyophilized pharmaceutical composition, comprising:
(i) a Clostridial toxin;
(ii) a lyoprotector selected from trehalose, sucrose, mannitol, sorbitol, glucose, and combinations thereof;
(iii) a surfactant selected from a poloxamer, a polysorbate and combinations thereof; and
(iv) at least two types of antioxidant selected from the list of chelating agents, chain terminators, and sacrificial antioxidants.

In this aspect of the invention, the clostridial toxin is preferably a botulinum toxin, the poloxamer is preferably poloxamer-188 and the polysorbate is preferably polysorbate-20 or 80. The chelating agents are preferably EDTA, EGTA or DTPA, the sacrificial antioxidant is preferably ascorbic acid, and the chain terminators are preferably methionine, cysteine, NAC, or BHT. As a result, the two types of antioxidants preferably include combinations of ascorbic acid with methionine, cysteine, NAC, or BHT. The two types of antioxidants also preferably include combinations of ascorbic acid with EDTA, EGTA or DTPA. The two types of antioxidants also preferably include the combinations of EDTA with methionine, cysteine, NAC, or BHT. The two types of antioxidant also preferably include the combinations of DTPA with methionine, cysteine, NAC, or BHT. The two types of antioxidant also preferably include the combinations of EGTA with methionine, cysteine, NAC, or BHT. In certain embodiments, the lyophilized composition is reconstituted with a tonicity agent selected from trehalose, sucrose, sodium chloride, mannitol, sorbitol, glucose, and combinations thereof. In at least one embodiment, the lyophilized composition is reconstituted with a reconstitution vehicle comprising NaCl prior to administration to a patient. In at least one embodiment, NaCl is present in an amount of 0.9% (w/w) in the reconstitution vehicle.

The invention also relates to a lyophilized pharmaceutical composition, comprising:
(i) a Clostridial toxin;
(ii) trehalose, sucrose, mannitol, sorbitol, or glucose;
(iii) a poloxamer;
(iv) a chelating agent; and
(v) a chain terminator.

In preferred embodiments, the chelating agent is EDTA, and the chain terminator is N-Acetyl-cysteine or methionine. The clostridial toxin is preferably a botulinum toxin and the poloxamer is preferably poloxamer-188.

The invention also relates to a lyophilized pharmaceutical composition, comprising:
(i) a Clostridial toxin;
(ii) trehalose, sucrose, mannitol, sorbitol, or glucose;
(iii) a poloxamer; and
(v) a chain terminator.

In preferred embodiments, the chain terminator is N-Acetyl-cysteine or methionine. The clostridial toxin is preferably a botulinum toxin and the poloxamer is preferably poloxamer-188.

The invention also relates to a liquid pharmaceutical composition, comprising:
(i) a Clostridial toxin;
(ii) a tonicity agent selected from trehalose, sucrose, sodium chloride, mannitol, sorbitol, glucose, and combinations thereof;
(iii) a surfactant selected from a poloxamer, a polysorbate, and combinations thereof; and
(iv) at least two types of antioxidant selected from the list of chelating agents, chain terminators, and sacrificial antioxidants.

In this aspect of the invention, the clostridial toxin is preferably a botulinum toxin, the poloxamer is preferably poloxamer-188 and the polysorbate is preferably polysorbate-20 or 80. The chelating agents are preferably EDTA, EGTA or DTPA, the sacrificial antioxidant is preferably ascorbic acid, and the chain terminators are preferably methionine, cysteine, NAC, or BHT. As a result, the two types of antioxidants preferably include combinations of ascorbic acid with methionine, cysteine, NAC, or BHT. The two types of antioxidants also preferably include combinations of ascorbic acid with EDTA, EGTA or DTPA. The two types of antioxidants also preferably include the combinations of EDTA with methionine, cysteine, NAC, or BHT. The two types of antioxidant also preferably include the combinations of DTPA with methionine, cysteine, NAC, or BHT. The two types of antioxidant also preferably include the combinations of EGTA with methionine, cysteine, NAC, or BHT.

### Methods of treatment

In embodiments, the invention provides methods of treating diseases, disorders, conditions, and the like, comprising the step of administering a pharmaceutical formulation of the invention to a subject in need thereof in an amount sufficient to produce improved patient function. In certain embodiments, the diseases are of a neuromuscular nature, such as, for example, those diseases that affect muscles and nerve control thereof, such as, for example, overactive bladder, and the like. In a preferred method, the compositions of the invention are used in the treatment of cardiac arrhythmia. Certain embodiments relate to the treatment of pain, such as, for example, treatment of headache pain, or back pain, or muscle pain, or the like. In certain embodiments, methods of the invention encompass the treatment of psychological disorders, including, for example, depression, anxiety, and the like. In a preferred method, the compositions of the invention are used in the treatment of depression.

Where the composition is used in the treatment of depression, it is preferably used to treat adults with moderate to severe major depressive disorder (MDD) either single episode or recurrent, wherein this diagnosis is based upon the Diagnostic and Statistical Manual of Mental Disorders Text Edition, published in 2000 (DSM-IV-TR). Any of the compositions of the invention can be used, but it is preferred that the lyophilized Botulinum toxin type A formulation describled in Table A is used, which is formulated in a solution for administration.

**Table A:**

| Raw Material (Ingredient) | Concentration, w/w | Quantity per vial | Function |
|---|---|---|---|
| Botulinum toxin type A | 1200 U/mL | 200U | Drug Substance |
| Trehalose | 80 mg/mL | 16 mg | Lyo Protectant |
| P188 | 40 mg/mL | 8 mg | Surfactant |
| L-methionine | 2 mg/mL | 0.4 mg | Antioxidant |
| L-Histidine | 1.430 mg/mL | 0.286 mg | Buffer |
| L-Histidine HCl, Monohydrate | 2.263 mg/mL | 0.453 mg | Buffer |
| Water | QS Adjust | QS Adjust | Solvent, removed by freeze-drying |

In one embodiment, the composition is reconstituted with a reconstitution vehicle comprising NaCl prior to administration to a patient. In one embodiment, NaCl may be present in an amount of 0.9% (w/w) in the reconstitution vehicle.

The composition is preferably administered in a single treatment session. The dosage is preferably a total dose of 30 Units or 50 Units. These dosages are preferably administered in divided injection into 6 (for the 30 units dosage) or 8 (for the 50 Units dosage) injection sites into the glabellar region of the forehead, preferably into the procerus and corrugator muscles.This treatment may be repeated in 3 to 6 months intervals. One advantage of the use of this method is that the patients treated with this composition show an improvement in the clinic Montgomery-Asberg Depression Rating scale. In addition, these patients also have improved clinic CGI-S scores (Clinical Global Impression of Change scores), and clinic HAM-D17 total scores (Hamilton Rating Scale for Depression).

Compositions and methods of the invention can be useful for the treatment, reduction of symptoms, and/or prevention of, for example, achalasia, anal fissure, anismus, blepharospasm, cerebral palsy, cervical dystonia, cervicogenic headache, hemifacial spasm, dyshidrotic eczema, dysphagia, dysphonia, esophageal dysmotility, esophageal muscular ring, esotropia (infantile), eyelift, facial myokemia, gait disturbances (idiopathic toe-walking), generalized dystonia, hemifacial spasm, hyperfunctional facial lines (glabellar, forehead, crows' feet, down-turned angles of the mouth), hyperhidrosis, incontinence (idiopathic or neurogenic), medication overuse headache, migraine headache, myoclonus, muscle mass or activity reduction, involving, for example, the masseter or the like, myofascial pain syndrome, obstructive urinary symptoms, pancreas divisum pancreatitis, Parkinson's disease, puborectalis syndrome, reduction of surgical scar tension, salivary hypersecretion, sialocele, sixth nerve palsy, spasticity, speech/voice disorders, strabismus, surgery adjunct (ophthalmic), tardive dyskinesia, temporomandibular joint disorders, tension headache, thoracic outlet syndrome, torsion dystonia, torticolis, Tourette's syndrome, tremor, whiplash-associated neck pain, pain, itching, inflammation, allergy, cancer and benign tumors, fever, obesity, infectious diseases, viral and bacterial, hypertension, cardiac arrhythmias, vasospasm, atherosclerosis, endothelial hyperplasia, venous thrombosis, varicose veins, apthous stomatitis, hypersalivation, temporomandibular joint syndrome, hyperhidrosis, bromhidrosis, acne, rosacea, hyperpigmention, hypertrophic scars, keloids, calluses and corns, skin wrinkling, excessive sebum production, psoriasis, dermatitis, allergic rhinitis, nasal congestion, post nasal drip, sneezing, ear wax, serous and suppurative otitis media, tonsil and adenoid hypertrophy, tinnitus, dizziness, vertigo, hoarseness, cough, sleep apnea, snoring, glaucoma, conjunctivitis, uveitis, strabismus, Grave's disease, excessive hair growth, hair loss, asthma, bronchitis, emphysema, mucus production, pleuritis, coagulation disorders, myeloproliferative disorders, disorders involving eosinophils, neutrophils, macrophages and lymphocytes, immune tolerance and transplantation, autoimmune disorders, dysphagia, acid reflux, hiatal hernia, gastritis and hyperacidity, diarrhea and constipation, hemorrhoids, urinary incontinence, prostatic hypertrophy, erectile dysfunction, priapism and Peyronie's disease, epididymitis, contraception, menstrual cramps, preventing premature delivery, endometriosis and fibroids, arthritis, osteoarthritis, rheumatoid, bursitis, tendonitis, tenosynovitis, fibromyalgia, seizure disorders, spasticity, headache, and neuralgias. In a preferred method, the compositions of the invention are used in the treatment of cardiac arrhythmia.

Where the composition is used in the treatment of cardiac arrhythmia, the patient is typically undergoing cardiac surgery. The formulation used in the treatment may be any of the compositions of the invention. However, the formulation is preferably based on the lyophilized formuation described in Table A, which is taken up into solution for administration.

**Table A:**

| Raw Material (Ingredient) | Concentration, w/w | Quantity per vial | Function |
|---|---|---|---|
| Botulinum toxin type A | 1200 U/mL | 200U | Drug Substance |
| Trehalose | 80 mg/mL | 16 mg | Lyo Protectant |
| P188 | 40 mg/mL | 8 mg | Surfactant |
| L-methionine | 2 mg/mL | 0.4 mg | Antioxidant |
| L-Histidine | 1.430 mg/mL | 0.286 mg | Buffer |
| L-Histidine HCl, Monohydrate | 2.263 mg/mL | 0.453 mg | Buffer |
| Water | QS Adjust | QS Adjust | Solvent, removed by freeze-drying |

In one embodiment, the composition is reconstituted with a reconstitution vehicle comprising NaCl prior to administration to a patient. In one embodiment, NaCl may be present in an amount of 0.9% (w/w) in the reconstitution vehicle.

The mode of administration of the composition is by one time injection into the major epicardial fat pads of the heart. The dosage used is 25 U per epicardial fat pad (125 U total dose) or 50 U per epicardial fat pad (250 U total dose). One advantage of the use of this method is that the incidence of atrial fibrillation (AF) as measured by ECG through week 4 can be reduced. Other advantages include: a reduction in the length of hospital stay; reduction in the length of stay in ICU; reduced rehospitalization rate of patients; reduced anticoagulant medication use; and reduced need for interventional procedures for Post Operative Atrial fibrillation (POAF), such as ablation, pacemaker implantation, electrical or pharmacologic cardioversion.

In certain embodiments, patients are limited to a maximum of 360U of botulinum toxin administered over any 90-day period.

### Treatment of nerve / muscle conditions

In an embodiment, the neuromuscular disease is hyperhidrosis. A subject suffering from hyperhidrosis, for example, receives about 59U per axilla, or about 58U per axilla, or about 57U per axilla, or about 56U per axilla, or about 55U per axilla, or about 54U per axilla, or about 53U per axilla, or about 52U per axilla, or about 51U per axilla, or about 50U per axilla, or about 49U per axilla, or about 48U per axilla, or about 47U per axilla, or about 46U per axilla, or about 45U per axilla, or about 44U per axilla, or about 43U per axilla, or about 42U per axilla, or about 41U per axilla, or about 40U per axilla, or about 39U per axilla, or about 38U per axilla, or about 37U per axilla, or about 36U per axilla, or less, per treatment of a pharmaceutical formulation of the present invention. In an embodiment, 50U total are injected intradermally into 10-15 sites spaced approximately 1-2cm apart.

In an embodiment, the neuromuscular disease is hemifacial spasm. A subject suffering from hemifacial spasm, for example receives between about 1.5 to 15U per treatment of the pharmaceutical formulation of the present invention. In a further example, the subject receives between about 1.5 to 3U, 1.5 to 5U, 1.5 to 7U, 1.5 to 10U, 1.5 to 12U, 1.5 to 15U, 5 to 10U, 5 to 15U, or 10 to 15U per treatment are administered to a patient with hemifacial spasm. In a still further example, the subject receives about 1.5U, about 2U, about 2.5U, about 3U, about 3.5U, about 4U, about 4.5U about 5U, about 5.5U, about 6U, about 6.5U, about 7U, about 7.5U, about 8U, about 8.5U, about 9U, about 9.5U, about 10U, about 10.5U, about 11U, about 11.5U, about 12U, about 12.5U, about 13U, about 13.5U, about 14U, about 14.5U, or about 15U per treatment are administered to a patient with hemifacial spasm. Dosages greater than 15U per treatment may also be administered to patients with hemifacial spasm to achieve a therapeutic response. A treatment session can comprise multiple treatments.

In an embodiment, the neuromuscular disease is cervical dystonia. A subject suffering from cervical dystonia, for example, receives between about 15 to 300U per treatment of a pharmaceutical formulation of the present invention. In a further example, the subject receives between about 35 to 250U, 65 to 200U, 85 to 175U, 105 to 160U, or 125 to 145U are administered to a patient with cervical dystonia. In an embodiment, dosages to the sternocleidomastoid muscle are limited to 100U or less. Dosages greater than 300U per treatment may also be administered to patients with cervical dystonia to achieve a therapeutic response. A treatment session can comprise multiple treatments.

In an embodiment, the neuromuscular disease is blepharospasm. A subject suffering from blepharospasm, for example, receives between about 1.25 to 2.5U of a pharmaceutical formulation of the present invention injected into the medial and lateral pretarsal orbicularis oculi of the upper lid and into the lateral pretarsal orbicularis oculi of the lower lid. In a further example, the subject receives about 1.5U, about 1.6U, about 1.7U, about 1.8U, about 1.9U, about 2.0U, about 2.1U, about 2.2U, about 2.3U, about 2.4U, about 2.5U, or more, per injection site. A treatment session can comprise multiple treatments.

In an embodiment, the neuromuscular disease is strabismus. A subject suffering from strabismus, for example, receives between about 1.25 to 2.5U per injection site of a pharmaceutical formulation of the present invention. In a further example, the subject recieves about 1.5U, , about 1.6U, about 1.7U, about 1.8U, about 1.9U, about 2.0U, about 2.1U, about 2.2U, about 2.3U, about 2.4U, about 2.5U, or more, per injection site to achieve a therapeutic response. In embodiments, lower doses are used for treatment of small deviations. In embodiments, vertical muscles and horizontal strabismus of less than 20 prism diameters can be treated with 1.25 to 2.5U per injection site. A treatment session can comprise multiple treatments.

In an embodiment, the neuromuscular disease is muscle spasticity. A subject suffering from muscle spasticity, for example, receives between about 20 to 200U per treatment of a pharmaceutical formulation of the present invention. In a further example, the subject receives between about 20 to 30U, 20 to 40U, 20 to 60U, 20 to 80U, 20 to 100U, 20 to 125U, 20 to 150U, or 20 to 175U per treatment are administered to a patient with muscle spasticity. In a still further example, the subject receives about 20U, about 25U, about 30U, about 35U, about 40U, about 45U, about 50U, about 55U, about 60U, about 65U, about 70U, about 75U, about 80U, about 85U, about 90U, about 95U, about 100U, about 105U, about 110U, about 115U, about 120U, about 125U, about 130U, about 135U, about 140U, about 145U, about 150U, about 155U, about 160U, about 165U, about 170U, about 175U, about 180U, about 185U, about 190U, about 195U, or about 200U per treatment are administered to a patient with muscle spasticity. In an embodiment, the biceps brachii can be injected with between 100U and 200U divided into 4 injection sites. In an embodiment, the flexor carpi radialis can be injected with between 12.5U and 50U in 1 injection site. In an embodiment, the flexor carpi ulnaris can be injected with between 12.5U and 50U in 1 injection site. In an embodiment, the flexor digitorum profundus can be injected with between 30U and 50U in one injection site. In an embodiment, the flexor digitorum sublimis can be injected with between 30U and 50 in a single injection site. Dosages greater than 200U per treatment may also be administered to patients with muscle spasticity to achieve a therapeutic response. A treatment session can comprise multiple treatments.

### Treatment of pain

In another embodiment, the present invention provides methods for treating pain comprising the step of administering a pharmaceutical formulation of the present invention to a subject in need thereof in an amount sufficient to reduce pain. In another embodiment, the patient suffers from myofascial pain, migraine headache pain, tension headache pain, neuropathic pain, facial pain, lower-back pain, sinus-headache pain, pain associated with temporomandibular joint disease, pain associated with spasticity or cervical dystonia, post-surgical wound pain, or neuralgia. A treatment session can comprise multiple treatments.

In an embodiment, the patient suffers from facial pain. A subject suffering from facial pain, for example, receives between about 4 to 40U per treatment of a pharmaceutical formulation of the present invention. In a further example, the subject receives between about 4 to 10U, 4 to 15U, 4 to 20U, 4 to 25U, 4 to 30U, 4 to 35U, 7 to 15U, 7 to 20U, 7 to 25U, 7 to 30U, 7 to 35U, or 7 to 40U per treatment are administered to a patient suffering from facial pain. In a still further example, the subject receives about 4U, about 5U, about 7.5U, about 10U, about 12.5U, about 15U, about 17.5U, about 20.0U, about 22.5U, about 25.0U, about 27.5U, about 30.0U, about 32.5U, about 35U, about 37.5U, or about 40U per treatment are administered to a patient with facial pain. Dosages greater than 40U per treatment may also be administered to patients with facial pain to achieve a therapeutic response. A treatment session can comprise multiple treatments.

In an embodiment, the patient suffers from myofascial pain. A subject suffering from myofascial pain, for example, receives between about 5 to 100U per treatment of a pharmaceutical formulation of the present invention. In a further example, the subject recieves between about 5 to 10U, 5 to 20U, 5 to 30U, 5 to 40 Units, 5 to 50 Units, 5 to 60 Units, 5 to 70 Units, 5 to 80 Units, 5 to 90U, 10 to 20U, 10 to 30U, 10 to 50U, or 10 to 60U, or 10 to 70U, or 10 to 80U, 10 to 90U, or 10 to 100U per treatment are administered to a patient suffering from myofascial pain. In a further example, the subject receives about 5U, about 10U, about 15U, about 20U, about 25U, about 30U, about 35U, about 40U, about 45U, about 50U, about 55U, about 60U, about 65U, about 70U, about 75U, about 80U, about 85U, about 90U, about 95U, or about 100U per treatment are administered to a patient with myofascial pain. Dosages greater than 100U per treatment may also be administered to patients with myofascial pain to achieve a therapeutic response. A treatment session can comprise multiple treatments.

In an embodiment, the subject suffers from lower-back pain. A subject suffering from lower-back pain, for example, receives between about 15 to 150U per treatment of a pharmaceutical formulation of the present invention. In a further example, the subject receives between about 15 to 30U, 15 to 50U, 15 to 75U, 15 to 100U, 15 to 125U, 15 to 150U, 20 to 100U, 20 to 150U, or 100 to 150U per treatment are administered to a patient with lower-back pain. In a still further example, the subject receives about 15U, about 20U, about 25U, about 30U, about 35U, about 40U, about 45U, about 50U, about 55U, about 60U, about 65U, about 70U, about 75U, about 80U, about 85U, about 90U, about 95U, about 100U, about 105U, about 110U, about 115U, about 120U, about 125U, about 130U, about 135U, about 140U, about 145U, or about 150U per treatment are administered to a patient with lower-back pain. Dosages greater than 150U per treatment may also be administered to patients with lower-back pain to achieve a therapeutic response. A treatment session can comprise multiple treatments.

In an embodiment, the patient suffers from migraine headache pain, including wherein the patient suffers from migraine headaches of 4 hours or more 15 or more days per month. A subject suffering from migraine-headache pain, for example, receives between about 0.5 to 200U per treatment of a pharmaceutical formulation of the present invention. In a further example, the subject receives between about 5 to 190U, 15 to 180U, 25 to 170U, 35 to 160U, 45 to 150U, 55 to 140U, 65 to 130U, 75 to 120U, 85 to 110U, or 95 to 105U per treatment are administered to a patient suffering from migraine-headache pain. A treatment session can comprise multiple treatments.

For example, about 0.5U, about 1.0U, about 1.5U, about 2.0U, about 2.5U, about 3.0U, about 3.5U, about 4.0U, about 4.5U, about 5.0U, about 5.5U, about 6.0U, about 6.5U, about 7.0U, about 7.5U, about 8.0U, about 8.5U, about 9.0U, about 9.5U, about 10.0U, about 12U, about 15U, about 17U, about 20U, about 22U, about 25U, about 27U, about 30U, about 32U, about 35U, about 37U, about 40U, about 42U, about 45U, about 47U, or about 50U per treatment site are administered to a patient with migraine-headache pain. A patient can be treated at multiple sites, such as, for example, 2 sites, 3 sites, 4 sites, 5 sites, 6 sites, 7 sites, 8 sites, 9 sites, 10 sites, 11 sites, 12 sites, 13 sites, 14 sites, 15 sites, 16 sites, 17 sites, 18 sites, 19 sites, 20 sites, 21 sites, 22 sites, 23 sites, 24 sites, 25 sites, 26 sites, 27 sites, 28 sites, 29 sites, 30 sites, 31 sites, 32 sites, or more, or the like. In an embodiment, a patient suffering from migraine is injected 31 times with 5U per 0.1mL injection, across the corrugator (2 injections of 5U each), procerus (1 injection of 5U), frontalis (4 injections of 5U each), temporalis (8 injections of 5U each), occipitalis (6 injections of 5U each), cervical paraspinal (4 injections of 5U each), and trapezius (6 injections of 5U each) muscles. With the exception of the procerus muscle which can be injected at the midline, all muscles can, in certain embodiments, be injected bilaterally with half of the injection sites to the left and half to the right side of the head and neck. Dosages greater than 200U per treatment may also be administered to patients with migraine-headache pain to achieve a therapeutic response. A treatment session can comprise multiple treatments.

In an embodiment, the patient suffers from sinus-headache pain. A subject suffering from sinus-headache pain, for example, receives between about 4 to 40U per treatment of a pharmaceutical formulation of the present invention. In a further example, the subject receives between about 4 to 10U, 4 to 15U, 4 to 20U, 4 to 25U, 4 to 30U, 4 to 35U, 7 to 15U, 7 to 20U, 7 to 25U, 7 to 30U, 7 to 35U, or 7 to 40U per treatment are administered to a patient suffering from sinus-headache pain. In a still further example, the subject receives about 4U, about 5U, about 7.5U, about 10U, about 12.5U, about 15U, about 17.5U, about 20.0U, about 22.5U, about 25.0U, about 27.5U, about 30.0U, about 32.5U, about 35U, about 37.5U, or about 40U per treatment are administered to a patient with sinus-headache pain. Dosages greater than 40U per treatment may also be administered to patients with sinus headache-pain to achieve a therapeutic response. A treatment session can comprise multiple treatments.

In an embodiment, the patient suffers from tension-headache pain. A subject suffering from tension-headache pain, for example, receives between about 5 to 50U per treatment of a pharmaceutical formulation of the present invention. In a further example, the subject receives between about 5 to 10U, 5 to 15U, 5 to 20U, 5 to 25U, 5 to 30U, 5 to 35U, 5 to 40U, 5 to 45U, 10 to 20U, 10 to 25U, 10 to 30U, 10 to 35U, 10 to 40U, or 10 to 45U per treatment are administered to a patient with tension-headache pain. In a still further example, the subject receives about 5U, about 10U, about 20U, about 25U, about 30U, about 35U, about 40U, about 45U, or about 50U per treatment are administered to a patient with tension-headache pain. In an embodiment, a patient suffering from tension headache is injected 31 times with 5U per 0.1mL injection, across the corrugator (2 injections of 5U each), procerus (1 injection of 5U), frontalis (4 injections of 5U each), temporalis (8 injections of 5U each), occipitalis (6 injections of 5U each), cervical paraspinal (4 injections of 5U each), and trapezius (6 injections of 5U each) muscles. With the exception of the procerus muscle which can be injected at the midline, all muscles can, in certain embodiments, be injected bilaterally with half of the injection sites to the left and half to the right side of the head and neck. Dosages greater than 200U per treatment may also be administered to patients with tension headache pain to achieve a therapeutic response. A treatment session can comprise multiple treatments.

In an embodiment, the patient suffers from sinus headache pain or facial pain associated with acute or recurrent chronic sinusitis. For example a pharmaceutical formulation of the present invention can be administered to the nasal mucosa or to the subcutaneous structures overlying the sinuses, wherein the administration of the formulation reduces the headache and/or facial pain associated with acute recurrent or chronic sinusitis. In further embodiments, any of the pharmaceutical formulations of the present invention can be administered to the nasal mucosa or to the subcutaneous structures overlying the sinuses, such as over one or more of the sinuses selected from the group consisting of: ethmoid; maxillary; mastoid; frontal; and sphenoid. In another embodiment, subcutaneous structures overlying the sinuses lie within one or more of the areas selected from the group consisting of: forehead; malar; temporal; post auricular; and lip. In embodiments, multiple injections of 5U each are administered to treat the sinus headache pain or facial pain associated with acute or recurrent chronic sinusitis.

In another embodiment, a patient suffering from sinus headache pain or facial pain associated with acute or recurrent chronic sinusitis is treated by administering any of the pharmaceutical formulations of the present invention to an afflicted area of the patient. In a further embodiment, the pharmaceutical formulations disclosed herein are administered to the projections of a trigeminal nerve innervating a sinus.

Patients suffering from sinus headache pain or facial pain associated with acute or recurrent chronic sinusitis often exhibit symptoms including rhinitis, sinus hypersecretion and/or purulent nasal discharge. In one embodiment, patients treated with the pharmaceutical formulations of the present invention exhibit symptoms of sinus hypersecretion and purulent nasal discharge.

Embodiments of the present invention also provide methods for treating a patient suffering from sinus headache pain or facial pain associated with acute or recurrent chronic sinusitis, wherein the subject suffers from neuralgia. In certain embodiments the neuralgia is trigeminal neuralgia. In another embodiment, the neuralgia is: associated with compressive forces on a sensory nerve; associated with intrinsic nerve damage, demyelinating disease, or a genetic disorder; associated with a metabolic disorder; associated with central neurologic vascular disease; or associated with trauma. In another embodiment of the present invention, the pain is associated with dental extraction or reconstruction.

### Treatment of urological disorders

In an embodiment, the invention also provide methods for treating a patient suffering from overactive bladder (OAB), such as, for example, that due to a neurologic condition (NOAB), or idiopathic OAB (IOAB). For example, pharmaceutical formulations of the present invention can be administered to the bladder or its vicinity, e.g. the detrusor, wherein the administration of the formulation reduces the urge incontinence associated with overactive bladder. In certain embodiments, the dosage can be, for example, 200U, or more, or less, or the like. For example, the dosage can be about 15U, about 20U, about 25U, about 30U, about 35U, about 40U, about 45U, about 50U, about 55U, about 60U, about 65U, about 70U, about 75U, about 80U, about 85U, about 90U, about 95U, about 100U, about 105U, about 110U, about 115U, about 120U, about 125U, about 130U, about 135U, about 140U, about 145U, about 150U, about 160U, about 170U, about 180U, about 190U, about 200U, about 210U, about 220, about 230U, about 240U, or more, or the like, per treatment. A patient can be injected at multiple sites, such as, for example, 2 sites, 3 sites, 4 sites, 5 sites, 6 sites, 7 sites, 8 sites, 9 sites, 10 sites, 11 sites, 12 sites, 13 sites, 14 sites, 15 sites, 16 sites, 17 sites, 18 sites, 19 sites, 20 sites, 21 sites, 22 sites, 23 sites, 24 sites, 25 sites, 26 sites, 27 sites, 28 sites, 29 sites, 30 sites, 31 sites, 32 sites, 33 sites, 34 sites, 35 sites, 36 sites, 37 sites, 38 sites, or more, or the like. In an embodiment, patients suffering from OAB are treated with 30 1mL injections of approximately 6.7U per injection into the detrusor muscle.

In an embodiment, the invention also provides methods for treating a patient suffering from neurogenic detrusor overactivity (NDO), such as that due to a neurologic condition. For example, pharmaceutical formulations of the present invention can be administered to the bladder or its vicinity, e.g. the detrusor, wherein the administration of the formulation reduces the urge incontinence associated with overactive bladder. In certain embodiments, the dosage can be, for example, 200U, or more, or less, or the like. For example, the dosage can be about 15U, about 20U, about 25U, about 30U, about 35U, about 40U, about 45U, about 50U, about 55U, about 60U, about 65U, about 70U, about 75U, about 80U, about 85U, about 90U, about 95U, about 100U, about 105U, about 110U, about 115U, about 120U, about 125U, about 130U, about 135U, about 140U, about 145U, about 150U, about 160U, about 170U, about 180U, about 190U, about 200U, about 210U, about 220, about 230U, about 240U, or more, or the like, per treatment. A patient can be injected at multiple sites, such as, for example, 2 sites, 3 sites, 4 sites, 5 sites, 6 sites, 7 sites, 8 sites, 9 sites, 10 sites, 11 sites, 12 sites, 13 sites, 14 sites, 15 sites, 16 sites, 17 sites, 18 sites, 19 sites, 20 sites, 21 sites, 22 sites, 23 sites, 24 sites, 25 sites, 26 sites, 27 sites, 28 sites, 29 sites, 30 sites, 31 sites, 32 sites, or more, or the like. In an embodiment, patients suffering from NDO are treated with 30 1mL injections of approximately 6.7U per injection into the detrusor muscle.

### Treatment of cosmetic features

In another embodiment, the present invention provides methods for cosmetically modifying soft-tissue features comprising the step of administering at least one pharmaceutical formulation of the present invention to a subject in need thereof in an amount sufficient to modify said features. In a further embodiment, the pharmaceutical formulation is administered via transcutaneous or transmucosal injection either at a single focus or multiple foci.

In embodiments, pharmaceutical formulations of the present invention are administered to the face or neck of the subject. In a further embodiment, the pharmaceutical formulations of the present invention are administered to the subject in an amount sufficient to reduce rhytides. For example, the formulation can be administered between eyebrows of the subject in an amount sufficient to reduce vertical lines between the eyebrows and on a bridge of a nose. The pharmaceutical formulations can also be administered near either one or both eyes of the subject in an amount sufficient to reduce lines at corners of the eyes. In an embodiment, compositions of the invention can be injected locally to smooth skin. In another embodiment, the pharmaceutical formulations of the present invention can also be administered to a forehead of the subject in an amount sufficient to reduce horizontal lines on said forehead. In yet another embodiment of the present invention the pharmaceutical formulation is administered to the neck of the subject in an amount sufficient to reduce muscle bands in the neck. In an embodiment, a pharmaceutical composition is applied to the masseter muscle to relax the muscle and / or decerase masseter mass.

In a further embodiment, the patient suffers from facial wrinkles. A subject suffering from facial wrinkles, for example, can receive between about 1 to 100U per treatment of a pharmaceutical formulation of the present invention. In a further example, the subject receives between about 1 to 10U, 1 to 20U, 1 to 30U, 1 to 40U, 1 to 50U, 1 to 60U, 1 to 70U, 1 to 80U, 1 to 90U, 5 to 20U, 5 to 30U, 5 to 40U, 5 to 50U, 5 to 60U, 5 to 70U, 5 to 80U, 5 to 90U, or 5 to 100U per treatment are administered to a patient with an inflammatory disorder. In a still further example, about 1U, about 10U, about 20U, about 30U, about 40U, about 50U, about 60U, about 70U, about 80U, about 90U, or about 100U per treatment are administered to a patient. Dosages greater than 100U per treatment may also be administered to patients suffering from inflammation or an inflammatory disorder to achieve a therapeutic response.

### Treatment of inflammation

In another embodiment, the present invention provides methods for treating inflammation comprising the step of administering a pharmaceutical formulation of the present invention to a subject in need thereof in an amount sufficient to reduce inflammation. In certain embodiments, pharmaceutical formulations of the present invention are administered to a patient without producing muscle weakness. In an embodiment, the pharmaceutical formulations of the present invention are administered to patients with an inflammatory condition. In certain embodiments the inflammatory condition is neurogenic inflammation. In another embodiment, the subject suffers from rheumatoid arthritis or a gastro-intestinal inflammatory disease.

In a further embodiment, the patient suffers from an inflammatory disorder. A subject suffering from an inflammatory disorder, for example, receives between about 1 to 100U per treatment of a pharmaceutical formulation of the present invention. In a further example, the subject receivesbetween about 1 to 10U, 1 to 20U, 1 to 30U, 1 to 40U, 1 to 50U, 1 to 60U, 1 to 70U, 1 to 80U, 1 to 90U, 5 to 20U, 5 to 30U, 5 to 40U, 5 to 50U, 5 to 60U, 5 to 70U, 5 to 80U, 5 to 90U, or 5 to 100U per treatment are administered to a patient with an inflammatory disorder. In a still further example, about 1U, about 10U, about 20U, about 30U, about 40U, about 50U, about 60U, about 70U, about 80U, about 90U, or about 100U per treatment are administered to a patient. Dosages greater than 100U per treatment may also be administered to patients suffering from inflammation or an inflammatory disorder to achieve a therapeutic response.

### Treatment of skin conditions

A method within the scope of the present invention for treating a skin disorder can have the step of local administration of a botulinum neurotoxin to a location of a skin disorder of a patient, such as to a face, hand or foot of a patient. The neurotoxin can be locally administered in an amount of between about 10-3 units/kg of patient weight and about 35 units/kg of patient weight. For example, the neurotoxin is locally administered in an amount of between about 10-2 U/kg and about 25 U/kg of patient weight. In a further example, the neurotoxin is administered in an amount of between about 10-1 U/kg and about 15 U/kg. In one method within the scope of the present invention, the neurotoxin is locally administered in an amount of between about 1 U/kg and about 10 U/kg. In a clinical setting it can be advantageous to administer from 1U to 3000U of a neurotoxin, such as botulinum toxin type A or B, to a skin disorder location by topical application or by subdermal administration, to effectively treat the skin disorder.

Administration of botulinum toxin can be carried out at multiple sites in the skin, wherein the sites of adjacent injections are separated by about 0.1 to 10cm, or about 0.5 to about 5cm, for example, by about 1.5 to about 3cm. The toxins may be any of the botulinum toxins A, B, C, D, E, F or G. The amounts administered may vary between 0.1 and 1000U, or about 1 to about 40, or from about 5 to about 10U, depending on the manufactures specifications, the class of the toxin and the mode of administration. The repeat time range for these administrations for maintenance of the desired change varies substantially according to the location of the injection, the condition to be adjusted and the condition of the patient. Thus the repeat time may vary from about 1 week to about 50 weeks, however a common range is about 4 to about 25 weeks, or even about 12 weeks to about 16 weeks.

The distances between administrations, for example, injections, can vary from about 1 mm to about 10cm, suitably from about 5mm to about 5cm, and more usually from about 1cm to about 3cm. Thus for example botulinum A may be suitably administered by intradermal injection between about 0.1 to about 10U at a separation of from about 0.5 to about 10cm.

In another embodiment, the present invention provides methods for treating cutaneous disorders comprising the step of administering a pharmaceutical formulation of the present invention to a subject in need thereof in an amount sufficient to reduce a sebaceous or mucous secretion. In further embodiments, the pharmaceutical formulations of the present invention are administered to a patient without producing muscle weakness. In certain embodiments the pharmaceutical formulations of the present invention are injected into one or more sites of an eyelid or conjunctiva. In another embodiment, the formulations of the present invention are administered to a body surface.

In another embodiment, the pharmaceutical formulations are administered in an amount sufficient to reduce cutaneous bacterial or fungal growth, including but not limited to Staphylococcus; Streptococcus and Moraxella. For example, the pharmaceutical formulations of the present invention are administered to an area selected from the group consisting of: eyelid; scalp; feet; groin; and armpit to reduce cutaneous infection.

### EXAMPLES

The following examples illustrate embodiments and aspects of the present invention and are not intended to limit the scope of the present invention.

### Example 1

### ACTIVITIES AND STABILITIES OF EXEMPLARY SOLID CLOSTRIDIAL PHARMACEUTICAL COMPOSITIONS RELATIVE TO PRIOR ART FORMULATIONS.

Bulk solutions of botulinum toxin were prepared by mixing an appropriate aliquot of a botulinum toxin type A with several vehicle solutions as described in the following Tables 1-3. The solutions were filled into glass vials and lyophilized using conventional freeze-drying conditions. Potency of the lyophilized formulations was tested by a cell based potency assay (CBPA) after reconstitution of the lyophiles with saline. Potency recovery results after freeze-drying and after storage at indicated temperatures are provided in the tables and normalized to target potency. The potencies of the solid compositions prepared according to aspects of the present invention were compared to three comparative formulations as shown in Tables 1-3.

**Table 1.1: Lyophilized formulations**

| | **Excipient, % w/w** | | | | | | | **Normalized potency, storage at 25 °C** | | |
|---|---|---|---|---|---|---|---|---|---|---|
| **Formulation** | **Treh** | **TWEEN^{®} 20** | **P 188** | **NaCl** | **Met** | **NAC** | **Buffer** | **T0** | **3 mo** | **6 mo** |
| Comparator 1 | 3 | 0.04 | | 0.9 | 0.2 | - | water | 80.6% | 86.4% | 80.8% |
| Comparator 2 | 2 | - | 4 | - | - | - | 20mM Histidine pH 5.5 | 81.5% | 68.6% | 68.0% |
| Formulation 1 | 2 | - | 4 | - | - | 0.03 | 20mM Histidine pH 5.5 | 98.6% | 91.9% | 86.9% |

| | | | | | | | | | | |
|---|---|---|---|---|---|---|---|---|---|---|
| Treh = trehalose; P 188 = poloxamer P 188; Met = L-methionine; NAC = N-acetyl-L-cysteine. | | | | | | | | | | |

**Table 1.2: Lyophilized formulations**

| | **Excipient, % w/w** | | | | | | | **Normalized potency, storage at 40 °C** | | |
|---|---|---|---|---|---|---|---|---|---|---|
| **Formulation** | **Treh** | **TWEEN ^{®}20** | **P 188** | **NaCl** | **Met** | **NA C** | **Buffer** | **1 mo** | **3 mo** | **6 mo** |
| Comparator 1 | 3 | 0.04 | | 0.9 | 0.2 | | Water | 74.8% | 70.3% | 24.0% |
| Comparator 2 | 2 | | 4 | | | | 20mM Histidine pH 5.5 | 64.8% | 57.8% | 46.8% |
| Formulation 1 | 2 | | 4 | | | 0.03 | 20mM Histidine pH 5.5 | 86.5% | 77.9% | 62.3% |

| | | | | | | | | | | |
|---|---|---|---|---|---|---|---|---|---|---|
| Treh = trehalose; P 188 = poloxamer P 188; Met = L-methionine; NAC = N-acetyl-L-cysteine. | | | | | | | | | | |

**Table 2: Lyophilized formulations**

| | **Excipient, % w/w** | | | | | | | | **Normalized potency (% of target)** | | |
|---|---|---|---|---|---|---|---|---|---|---|---|
| **Formulation** | Treh | Sucr | TWEEN^{®} 20 20 | P188 | NaCl | Met | NAC | **Buffer** | **T0** | **1 mo -20 °C** | **1 mo 40 °C** |
| Comparator 2 | 2 | | | 4 | | | | 20mM Histidine pH 5.5 | 87.15% | 88.95% | 76.32% |
| Comparator 3 | | 3 | 0.04 | | 0.9 | 0.2 | | Water | 84.06% | 85.18% | 72.86% |
| Formulation 2 | 2 | | | 4 | | 0.2 | | 20mM Histidine pH 6.0 | 98.60% | 120.85% | 91.17% |
| Formulation 3 | 8 | | 0.04 | | | | 0.03 | 20mM Histidine pH 6.0 | 105.2% | 110.05% | 96.89% |

| | | | | | | | | | | | |
|---|---|---|---|---|---|---|---|---|---|---|---|
| Treh = trehalose; Sucr = sucrose; P188 = poloxamer P 188; Met = L-methionine; NAC = N-acetyl-L-cysteine. | | | | | | | | | | | |

**Table 3: Lyophilized formulations**

| | **Excipient, % w/w** | | | | | | | | **Normalized potency, storage at 25 °C** | | |
|---|---|---|---|---|---|---|---|---|---|---|---|
| **Formulation** | Treh | Sucr | TWEEN^{®} 20 20 | P18 8 | NaCl | Met | NAC | **Buffer** | **T0** | **3 mo** | **7.5 mo** |
| Comparator 2 | 2 | | | 4 | | | | 20mM Histidine, pH 5.5 | 87.15 % | 78.0% | 78.0% |
| Formulation 2 | 2 | | | 4 | | 0.2 | | 20mM Histidine, pH 6.0 | 98.6% | 97.0% | 98.0% |
| Formulation 4 | 8 | | | 0.6 | | 0.2 | | 20mM Histidine, pH 6.0 | 86.48 % | 83.0% | 84.0% |

| | | | | | | | | | | | |
|---|---|---|---|---|---|---|---|---|---|---|---|
| P-188 = Poloxamer P-188; Met = L-methionine. | | | | | | | | | | | |

### Example 2

### ACTIVITIES OF LIQUID CLOSTRIDIAL PHARMACEUTICAL COMPOSITIONS IN THE PRESENCE OR ABSENCE OF ANTIOXIDANTS

Bulk drug product solutions were prepared by mixing an appropriate aliquot of a botulinum toxin type A with three different vehicle solutions as shown in Table 4. All three formulations contained 8% w/w trehalose, 4% w/w P188 and 20 mM Histidine buffer at pH 6.0. The target potency was 100units/mL. Formulation 10 contained no antioxidant. Formulations 11 and 12 contained NAC and methionine, respectively. The bulk solutions were filled into 2 mL glass vials (1.25 mL fill), and sealed with rubber stopper and aluminum shell. Potency of the formulations was tested by a cell based potency assay (CBPA) after filling (time zero, t0) and storage for one month at four temperatures (-70, 5, 25, and 40°C). Potency test results are given in Table 4. Briefly, methionine-containing formulation (#12) retained its potency after one month storage at all four temperatures, including 40°C, whereas the formulation w/o an antioxidant lost approx. 17 % potency at 25°C and lost essentially all activity (i.e., complete inactivation) at 40°C. A second antioxidant tested, N-acetyl-L-cysteine, accelerated loss of potency as compared with the antioxidant-free formulation, demonstrating that N-acetyl-L-cysteine acted as pro-oxidant in this formulation. The liquid composition comprising NAC lost over 50% potency after storage for 1 month at 25 °C and lost essentially all activity (i.e., complete inactivation of the toxin) after storage for 1 month at 40 °C. In contrast, as shown in Table 1, the lyophilized compositions comprising NAC lost 7% ((98.6%-91.9%)/98.6%) potency after storage for 3 months at 25 °C and lost 12% ((98.6%-86.5%)/98.6%) potency after storage for 1 month at 40 °C. This demonstrates that NAC can function as a stabilizer in the lyophilized compositions.

**Table 4: Liquid formulations**

| | | **Potency, U/mL** | | | | |
|---|---|---|---|---|---|---|
| **Formulation No.*** | **Antioxidant** | **T0** | **1 mo. -70°C** | **1 mo. 5°C** | **1 mo. 25°C** | **1 mo. 40°C** |
| Formulation 10 | none | 128 | 135 | 135 | 106 | 0.225 |
| Formulation 11 | N-acetyl-L-cysteine, 0.2 % w/w | 128 | 133 | 129 | 61 | 0.2 |
| Formulation 12 | L-methionine, 0.2% w/w | 133 | 146 | 146 | 145 | 138 |

| | | | | | | |
|---|---|---|---|---|---|---|
| *Each formulation contained the same amount of botulinum toxin, 8 w/w% trehalose, and 4 w/w% poloxamer P188 in histidine buffer. | | | | | | |

### Example 3

### IMPACT OF EXEMPLARY ANTIOXIDANTS ON THE STABILITY OF EXEMPLARY LIQUID FORMULATIONS.

Bulk drug product solutions were prepared by mixing an appropriate aliquot of a botulinum toxin type A with different antioxidants as shown in Table 5. All formulations contained 8% w/w trehalose, 4% w/w P188, 20 mM Histidine buffer at pH 6.0 and one or more antioxidants as listed. Target potency was 100 U/mL. The bulk solutions were filled into 2 mL glass vials (1.25 mL fill), and sealed with rubber stopper and aluminum shell. Potency of the formulations was tested by a cell based potency assay (CBPA) after filling (time zero, t0) and storage for two weeks and 1 month at 40°C. Potency test results are given in Table 5. Liquid compositions comprising NAC and EDTA sodium salt (Formulation 26) retained full potency after storage at 40 °C for 2 weeks and 1 month, as did a composition comprising NAC, EDTA sodium salt and tryptophan (Formulation 27). In contrast, as shown in Table 5, liquid compositions comprising NAC but not EDTA (Formulation 25) lost essentially all potency after 2 weeks storage at 40 °C. This demonstrates that the combination of antioxidants - a chelating agent (e.g., EDTA, EGTA or DTPA) and/or a chain terminator antioxidant (e.g., methionine, cysteine, NAC and BHT) provides a stabilizing effect on the botulinum toxin.

**Table 5: Liquid formulations**

| | **Antioxidant²** | | | | | | | **Potency U/mL** | | |
|---|---|---|---|---|---|---|---|---|---|---|
| **Formulatio n No.¹** | NAC % | Met % | TRP % | GSH % | NaSu 1 % | PrpGa 1 % | EDT A % | T0 | 2 wks 40 °C | 1 mo. 40 °C |
| Formulation 20 | | 0.2 | | | | | | 126 | 129 | 130 |
| Formulation 21 | | | 0.2 | | | | | 127 | 13.71 | NT³ |
| Formulation 22 | | | | 0.2 | | | | 123 | 3.76 | NT |
| Formulation 23 | | | | | 0.2 | | | 23.7 | 0.161 | NT |
| Formulation 24 | | | | | | 0.2 | | 0.164 | 0.150 | NT |
| Formulation 25 | 0.2 | | 0.2 | | | | | 133 | 0.253 | NT |
| Formulation 26 | 0.2 | | | | | | 0.03 | 129 | 127 | 127 |
| Formulation 27 | 0.2 | | 0.2 | | | | 0.03 | 129 | 125 | 122 |
| Formulation 28 | | | 0.2 | 0.2 | | | | 126 | 2.45 | NT |

| | | | | | | | | | | |
|---|---|---|---|---|---|---|---|---|---|---|
| ¹ Each formulation contained 100 U/mL botulinum toxin, 8 w/w% trehalose, and 4 w/w% poloxamer P188 in 20 mM histidine buffer, pH 6.0 and the specified antioxidant. ² NAC = N-acetyl-L-cysteine; Met = L-methionine; TRP = L-tryptophan; GSH = L-glutathione; NaSul = sodium sulfite; PrpGal = propyl gallate; EDTA = ethylene diamine tetraacetic acid, sodium salt. ³ NT=not tested | | | | | | | | | | |

### Example 4

### IMPACT OF CHOICE OF SURFACTANT ON THE STABILITY OF EXEMPLARY LIQUID COMPOSITIONS

Bulk drug product solutions were prepared by mixing an appropriate aliquot of a botulinum toxin type A with a poloxamer or a polysorbate as shown in Table 6. All formulations contained 8% w/w trehalose, 0.2% w/w methionine in 20 mM Histidine buffer at pH 6.0 and 4% w/w P188 or 0.04% w/w Tween-20 as listed. Target potency was 100 U/mL. The bulk solutions were filled into 2 mL glass vials (1.25 mL fill), and sealed with rubber stopper and aluminum shell. Potency of the formulations was tested by a cell based potency assay (CBPA) after filling to give an initial potency. Potency was measured again after storage for 1 month at 40°C. Potency test results are given in Table 6. As is evident from the results in table 6, the liquid formulation comprising poloxamer provided improved stability after storage at 40°C for one month relative to the corresponding formulation comprising polysorbate.

**Table 6: Liquid formulations**

| Formulation no. | Tre | Tw-20 | P188 | Met | Buffer | Storage time (Months) | Storage Temp. | CBPA (U/mL) Initial | CBPA (U/mL) 1 month |
|---|---|---|---|---|---|---|---|---|---|
| 29 | 8 | 0 | 4 | 0.2 | 20 mM His pH 6.0 | 1 | 40°C | 133 | 138 |
| 30 | 8 | 0.04 | 0 | 0.2 | 20 mM His, pH 6.0 | 1 | 40°C | 114 | 9 |

| | | | | | | | | | |
|---|---|---|---|---|---|---|---|---|---|
| Tre = Trehalose; P-188 = Poloxamer-188; Tw-20 = Tween-20; Met = L-Methionine; His = L-Histidine. CBPA gives the residual activity expressed in U/ml | | | | | | | | | |

### Example 5

### IMPACT OF ABSENCE OF TONICITY AGENT ON THE STABILITY OF EXEMPLARY LIQUID COMPOSITIONS

Bulk drug product solutions were prepared by mixing an appropriate aliquot of a botulinum toxin type A with different components as shown in Tables 7 and 8. The amounts of components contained were 0% or 8% w/w trehalose, 0% or 0.2% w/w methionine, 0% or 4% w/w P188, all of which were formulated in 20 mM Histidine buffer at pH 6.0. Target potency was 100, 150, or 200 U/mL. The bulk solutions were filled into 2 mL glass vials (1.25 mL fill), and sealed with rubber stopper and aluminum shell. Potency of the formulations was tested by a cell based potency assay (CBPA) after filling to give an initial potency. Potency was measured again after storage for 1 month at -70°C and 40°C. Potency test results are given in Tables 7 and 8. As is evident from the results in these tables, liquid formulations comprising poloxamer and no tonicity agent provide excellent stability after storage for one month at -70°C and 40°C. This is supported by the results in table 10 below for formulation 40. Meanwhile, liquid formulations comprising polysorbate and no tonicity agent provide some stability after storage for one month at -70°C.

**Table 7: Liquid formulations**

| Formulation no. | Formulation composition (% w/v) | | | | CBPA, U/mL | CBPA, U/mL | CBPA, U/mL | CBPA, U/mL |
|---|---|---|---|---|---|---|---|---|
| | trehalose | P188 | Met | Buffer | target | Initial (U/ml) | 1 month, -70°C | 1 month, 40°C |
| 31 | 8 | 4 | 0 | 20 mM His pH 6.0 | 100 | 128 | 135 | 0.225 |
| 32 | | | 0.2 | 20mM His pH 6.0 | 150 | 52 | 13 | 0 |
| 33 | 8 | | 0.2 | 20mM His pH 6.0 | 150 | 131 | 145 | 30 |
| 34 | | 4 | 0.2 | 20mM His pH 6.0 | 100 | 128 | 142 | 103 |
| 35 | 8 | 4 | 0.2 | 20mM His pH 6.0 | 100 | 126 | | 130 |

**Table 8: Liquid formulations**

| **Formulation No.** | **Tre** | **Sucr** | **Tw-20** | **P188** | **Met** | **Buffer** | **Target potency (U/mL)** | **CBPA (U/ml) -70 °C** | **CBPA (U/ml) 40 °C,** |
|---|---|---|---|---|---|---|---|---|---|
| 36 | 0 | 0 | 0.04 | 0 | 0.2 | 20 mM His pH 6.0 | 100 | 25 | 1 |
| 37 | 0 | 0 | 0 | 4 | 0.2 | 20 mM His pH 6.0 | 100 | 105 | 72 |

| | | | | | | | | | |
|---|---|---|---|---|---|---|---|---|---|
| Sucr = sucrose; Tre = Trehalose; P-188 = Poloxamer-188; Tw-20 = Tween-20; Met = L-Methionine; His = L-Histidine. Initial = initial activity expressed in U/ml Results after 1 month are the residual activity expressed in U/ml | | | | | | | | | |

### Example 6

### STABILITY OF VARIOUS LIQUID COMPOSITIONS

Bulk drug product solutions were prepared by mixing an appropriate aliquot of a botulinum toxin type A with vehicle solutions, containing different stabilizers and surfactants as shown in Table 9. All formulations contained 8% or 0% w/w trehalose or sucrose, 4% w/w P188 or 0.04% w/w PS-20, optionally 20 mM Histidine buffer at pH 6.0, 0.2% w/w methionine, and optionally 0.9% w/w NaCl. Target potency was 100 U/mL. The bulk solutions were filled into 2 mL glass vials (1.25 mL fill), and sealed with rubber stopper and aluminum shell. Potency of the formulations was tested by cell based potency assay (CBPA) after filling (time zero, t0) and storage at 5°C. Potency test results are given in Table 10.

**Table 9: Liquid formulations**

| Formulation No. | Target potency U/mL | Tre % | Suc % | NaCl % | P-188 % | PS-20 % | Met % | Buffer |
|---|---|---|---|---|---|---|---|---|
| 38 | 100 | | 8 | | 4 | | 0.2 | 20 mM His, pH 6.0 |
| 39 | 100 | | 8 | | 4 | | 0.2 | Water |
| 40 | 100 | | | 0.9 | 4 | | 0.2 | 20 mM His, pH 6.0 |
| 41 | 100 | 8 | | | | 0.04 | 0.2 | 20 mM His, pH 6.0 |

| | | | | | | | | |
|---|---|---|---|---|---|---|---|---|
| Suc = sucrose; Tre = Trehalose; P-188 = Poloxamer-188; Ps-20 = Tween-20; Met = L-Methionine; His = L-Histidine. | | | | | | | | |

**Table 10: Liquid formulations**

| Formulation No. | Storage Temperature | 0 months | 5.5 months | 7.5 months | 12 months |
|---|---|---|---|---|---|
| 38 | 5 °C | 117 | 88 | NT | 94 |
| 39 | 5 °C | 114 | NT | 108 | 91 |
| 40 | 5 °C | 94 | NT | 80 | 62 |
| 41 | 5 °C | 114 | NT | 117 | 91 |

| | | | | | |
|---|---|---|---|---|---|
| Results are the residual activity expressed in U/ml | | | | | |

### Example 7

### IMPACT OF CHOICE OF SUGAR ON STABILITY OF EXEMPLARY LYOPHILIZED FORMULATIONS

Bulk solutions of botulinum toxin were prepared by mixing an appropriate aliquot of a botulinum toxin type A with several vehicle solutions as described in Table 11. The amounts of components contained were 0% or 8% w/w trehalose or sucrose, 0.2% w/w methionine, 0% or 4% w/w P188, 0% or 0.04% w/w Tween 20 all of which were formulated in 20 mM Histidine buffer at pH 6.0. Target potency was 200 U/mL. These amounts are shown in Table 11 below. The solutions were filled into glass vials and lyophilized using conventional freeze-drying conditions. Potency of the lyophilized formulations was tested by a cell based potency assay (CBPA) after reconstitution of the lyophilized compositions after storage for 2 weeks at -70°C or 40°C. Potency recovery results are given in Table 12. By comparing Formulation No. 42 with Formulation No. 44 and Formulation No. 43 with Formulation No. 45, the impact of the type of sugar on these lyophilized formuations can be derived. The potency of the botulinum toxin in Formulation 42 and in Formulation 44 after 2 weeks of storage at -20 °C and at 40 °C reveals a higher potency recovery was achieved in lyophilized compositions comprising trehalose, as compared to lyophilized compositions comprising sucrose.

**Table 11: Lyophilized formulations**

| **Formualtion No.** | **Tre** | **Sucr** | **Tw-20** | **P188** | **Met** | **Buffer** | **Target potency (U/vial)** |
|---|---|---|---|---|---|---|---|
| 42 | 8 | 0 | 0 | 4 | 0.2 | 20 mM His pH 6.0 | 200 |
| 43 | 8 | 0 | 0.04 | 0 | 0.2 | 20 mM His pH 6.0 | 200 |
| 44 | 0 | 8 | 0 | 4 | 0.2 | 20 mM His pH 6.0 | 200 |
| 45 | 0 | 8 | 0.04 | 0 | 0.2 | 20 mM His pH 6.0 | 200 |

| | | | | | | | |
|---|---|---|---|---|---|---|---|
| Tre = Trehalose; Sucr = Sucrose; Tw-20 = Tween-20; P-188 = Poloxamer-188; Met = L-Methionine; His = L-Histidine. | | | | | | | |

**Table 12: Lyophilized formulations**

| **Formulation No.** | **-20 °C, 2 Weeks** | **40 °C, 2 Weeks** |
|---|---|---|
| 42 | 166 | 162 |
| 43 | 147 | 137 |
| 44 | 149 | 134 |
| 45 | 150 | 142 |

### Example 8

### IMPACT OF CHOICE OF SURFACTANT ON THE STABILITY OF EXEMPLARY LYOPHILIZED COMPOSITIONS

The results in Tables 11 and 12 also demonstrate the impact of the choice of surfactant on the stability of certain lyophilized compositions. By comparing Formulation No. 42 with Formulation No. 43 and Formulation No. 44 with Formulation No. 45, the impact of the type of surfactant on these lyophilized formuations can be derived. The potency of the botulinum toxin in Formulation 43 and in Formulation 45 after 2 weeks of storage at -20 °C and at 40 °C reveals a higher potency recovery was achieved in lyophilized compositions comprising a poloxamer surfactant, as compared to lyophilized compositions comprising a polysorbate surfactant.

### Example 9

### TREATMENT OF DEPRESSION WITH EXEMPLARY COMPOSITIONS

A 58-year-old college professor presents symptoms of depression. Her physician diagnoses recurrent moderate to severe major depressive disorder (MDD) based upon the DSM-IV-TR criteria. The physician administers by intramuscular injection a botulinum toxin solution made using the composition shown in Table A reconstituted in saline into the procerus and corrugator muscles in a single treatment session. 30 units in total are administered at 6 injection sites. The procerus and corrugator muscles are paralysed. It is later confirmed using the Montgomery-Asberg Depression Rating scale that the treatment has alleviated the depression. Additional efficacy measures including the clinic CGI-S score (Clinical Global Impression of Change scores), and the clinic HAM-D17 total score (Hamilton Rating Scale for Depression) further confirm this. The treatment is repeated after 3 months.

### Example 10

### TREATMENT OF DEPRESSION WITH EXEMPLARY COMPOSITIONS

A 21-year-old student presents symptoms of depression. His physician diagnoses single episode moderate to severe major depressive disorder (MDD) based upon the DSM-IV-TR criteria. The physician administers by intramuscular injection a botulinum toxin solution made using the composition shown in Table A reconstituted in saline to the procerus and corrugator muscles in a single treatment session. 50 units in total are administered at 8 injection sites. The procerus and corrugator muscles are paralysed. It is later confirmed using the Montgomery-Asberg Depression Rating scale that the treatment has alleviated the depression. Additional efficacy measures including the clinic CGI-S score (Clinical Global Impression of Change scores), and the clinic HAM-D17 total score (Hamilton Rating Scale for Depression) further confirm this. The treatment is repeated after 6 months.

### Example 11

### TREATMENT OF CARDIAC ARRHYTHMIA WITH EXEMPLARY COMPOSITIONS

A 41-year-old teacher undergoing cardiac surgery shows symptoms of cardiac arrhythmia. The surgeon administers a botulinum toxin solution made using the composition shown in Table A reconstituted in saline into the major epicardial fat pads of the heart of the patient. 50 U is administered per epicardial fat pad, up to a total of 250 U. The patient is followed by ECG for a four week period following the injection. The indcidence of atrial fibrillation is reduced. In addition, the following improvements are noted for this patient: the length of hospital stay is reduced, length of stay in ICU is reduced, anticoagulant medication use is reduced, and the need for interventional procedures for Post Operative Atrial fibrillation(POAF) such as ablation, pacemaker implantation, electrical or pharmacologic cardioversion are reduced.

### Example 12: treatment of cardiac arrhythmia with exemplary compositions.

A 66-year-old pensioner undergoing cardiac surgery shows symptoms of cardiac arrhythmia. The surgeon administers a botulinum toxin solution made using the composition shown in Table A reconstituted in saline into the major epicardial fat pads of the heart of the patient. 25 U is administered per epicardial fat pad, up to a total of 125 U. The patient is followed by ECG for a four week period following the injection. The indcidence of atrial fibrillation is reduced. In addition, the following improvements are noted for this patient: the length of hospital stay is reduced, length of stay in ICU is reduced, anticoagulant medication use is reduced, and the need for interventional procedures for Post Operative Atrial fibrillation(POAF) such as ablation, pacemaker implantation, electrical or pharmacologic cardioversion are reduced.

Many alterations and modifications may be made by those having ordinary skill in the art, without departing from the spirit and scope of the disclosure. Therefore, it must be understood that the described embodiments have been set forth only for the purposes of examples, and that the embodiments should not be taken as limiting the scope of the invention. The following articles and items of the invention are, therefore, to be read to include not only the combination of elements which are literally set forth, but all equivalent elements for performing substantially the same function in substantially the same way to obtain substantially the same result. The invention is thus to be understood to include those that have been described above, those that are conceptually equivalent, and those that incorporate the ideas of the disclosure.

The present disclosure also includes the following set of articles:
1. A pharmaceutical composition comprising:
(i) a Clostridial toxin active ingredient;
(ii) a tonicity agent and/or a lyoprotector;
(iii) a poloxamer and/or a polysorbate; and
(iv) an antioxidant.
2. The composition according to article 1, comprising botulinum toxin.
3. The composition according to article 1 or article 2, comprising trehalose.
4. The composition according to any one of articles 1 to 3, comprising poloxamer 188 and/or polysorbate 20.
5. The composition according to any one of articles 1 to 4, comprising one or more of methionine and N-Acetyl-cysteine.
6. The composition according to any one of articles 1 to 5, comprising botulinum toxin, trehalose, one of poloxamer 188 or polysorbate 20, and one of methionine or N-Acetyl-cysteine.
7. The composition according to any one of articles 1 to 6, comprising botulinum toxin, trehalose, poloxamer 188, and methionine.
8. The composition according to article 7, wherein the relative weight amounts (%, w/w) of trehalose, poloxamer 188, and methionine are within the following ranges:

| | |
|---|---|
| trehalose | 1to 10 |
| poloxamer 188 | 0.5 to 5 |
| methionine | 0.1 to 0.3 |

9. The composition according to any one of articles 1 to 6, comprising botulinum toxin, trehalose, polysorbate 20, and methionine.
10. The composition according to article 9, wherein the relative weight amounts (%, w/w) of trehalose, polysorbate 20, and methionine are within the following ranges:

| | |
|---|---|
| trehalose | 1to 10 |
| polysorbate 20 | 0.02 to 0.06 |
| methionine | 0.1 to 0.3 |

11. The composition according to any one of articles 1 to 6, comprising botulinum toxin, trehalose, poloxamer 188, and N-Acetyl-cysteine.
12. The composition according to article 11, wherein the relative weight amounts (%, w/w) of trehalose, poloxamer 188, and N-Acetyl-cysteine are within the following ranges:

| | |
|---|---|
| trehalose | 1to 10 |
| poloxamer 188 | 0.5 to 5 |
| N-Acetyl-cysteine | 0.01 to 0.5 |

13. The composition according to any one of articles 1 to 6, comprising botulinum toxin, trehalose, polysorbate 20, and N-Acetyl-cysteine.
14. The composition according to 13, wherein the relative weight amounts (%, w/w) of trehalose, polysorbate 20, and N-Acetyl-cysteine are within the following ranges:

| | |
|---|---|
| trehalose | 1to 10 |
| polysorbate 20 | 0.02 to 0.06 |
| N-Acetyl-cysteine | 0.01 to 0.5 |

15. The composition according to any one of articles 1 to 14, comprising histidine.
16. The composition according to any one of articles 1 to 15, comprising no animal-derived protein.
17. The composition according to any one of articles 1 to 4; comprising ethylene diamine tetraacetic acid sodium salt (EDTA) or an EDTA analog.
18. The composition according to any one of articles 5 and 6, further comprising ethylene diamine tetraacetic acid sodium salt (EDTA) or an EDTA analog.
19. The composition according to any one of articles 11 to 14, further comprising ethylene diamine tetraacetic acid sodium salt (EDTA) or an EDTA analog.
20. The composition according to any one of articles 1-19, whereint the relative weight amount (%, w/w) of EDTA ranges from about 0.01 to 0.10.
21. The composition according to any one of articles 1 to 20, wherein the composition is a solid formulation.
22. The composition according to any one of articles 12, 14, 19 and 20, wherein the composition is a solid formulation and the relative weight amount (%, w/w) of N-Acetyl-cysteine is 0.01 to 0.05.
23. The composition according to any one of articles 1 to 20, wherein the composition is a liquid formulation and has a pH of from 5 to 7.
24. The composition according to any one of articles 11 to 14, 19 and 20, wherein the composition is a liquid formulation and has a pH of from 5 to 7, wherein the relative weight amount of N-Acetyl-cysteine is 0.1 to 0.5.

The present disclosure also includes the following set of embodiments.
1. A pharmaceutical composition comprising:
(i) a Clostridial toxin active ingredient;
(ii) a tonicity agent and/or a lyoprotector;
(iii) a poloxamer and/or a polysorbate; and
(iv) an antioxidant.
2. The composition according to embodiment 1, comprising botulinum toxin.
3. The composition according to embodiment 1 or embodiment 2, comprising trehalose or sucrose.
4. The composition according to any one of embodiments 1 to 3, comprising poloxamer 188 and/or polysorbate 20.
5. The composition according to any one of embodiments 1 to 4, comprising one or more of methionine and N-Acetyl-cysteine.
6. The composition according to any one of embodiments 1 to 5, comprising botulinum toxin, trehalose or sucrose, one of poloxamer 188 or polysorbate 20, and one of methionine or N-Acetyl-cysteine.
7. The composition according to any one of embodiments 1 to 6, comprising botulinum toxin, trehalose or sucrose, poloxamer 188, and methionine.
8. The composition according to embodiment 7, wherein the relative weight amounts (%, w/w) of trehalose or sucrose, poloxamer 188, and methionine are within the following ranges:

| | |
|---|---|
| trehalose or sucrose | 1to 10 |
| poloxamer 188 | 0.5 to 5 |
| methionine | 0.1 to 0.3. |

9. The composition according to embodiment 8, wherein the relative weight amounts (%, w/w) of trehalose or sucrose, poloxamer 188, and methionine are:

| a) | trehalose or sucrose | 7 to 9 |
|---|---|---|
| | poloxamer 188 | 3.5 to 4.5 |
| | methionine | 0.15 to 0.25; |
| and are preferably | trehalose or sucrose | 8 |
| | poloxamer 188 | 4 |
| | methionine | 0.2; or |

| b) | trehalose or sucrose | 7 to 9 |
|---|---|---|
| | poloxamer 188 | 0.5 to 0.7 |
| | methionine | 0.15 to 0.25; |
| and are preferably | trehalose or sucrose | 8 |
| | poloxamer 188 | 0.6 |
| | methionine | 0.2; or |

| c) | trehalose or sucrose | 1 to 3 |
|---|---|---|
| | poloxamer 188 | 3.5 to 4.5 |
| | methionine | 0.15 to 0.25 |
| | NaCl | 0.4 to 0.8; |
| and are preferably | trehalose or sucrose | 2 |
| | poloxamer 188 | 4 |
| | methionine | 0.2 |
| | NaCl | 0.6; or |

| d) | trehalose or sucrose | 7 to 9 |
|---|---|---|
| | poloxamer 188 | 3.5 to 4.5 |
| | methionine | 0.15 to 0.25 |
| | EDTA | 0.01 to 0.05; |
| and are preferably | trehalose or sucrose | 8 |
| | poloxamer 188 | 4 |
| | methionine | 0.2 |
| | EDTA | 0.03. |

10. The composition according to any one of embodiments 7 - 9, wherein the composition is a liquid composition, wherein the composition preferably comprises a buffer.
11. The composition according to embodiment 10, wherein the buffer is histidine, wherein the concentration of histidine is preferably 20 mM, wherein the pH is preferably in the range 5-7, and wherein the pH is more preferably in the range 5.5-6.5, and the pH is most preferably in the range 5.5-6.
12. The composition according to embodiment 10, wherein the composition consists of trehalose or sucrose, poloxamer 188, methionine, a buffer, and optionally NaCl, and optionally EDTA, wherein the buffer is preferably histidine, wherein the pH is preferably in the range 5-7, the pH is more preferably in the range 5.5-6.5, and wherein the pH is most preferably in the range 5.5-6.
13. The composition according to any one of embodiments 7 - 9, wherein the composition is a solid composition, wherein the composition is preferably lyophilised.
14. The composition according to embodiment 13, further comprising a buffer, wherein the buffer is preferably histidine, wherein the histidine is preferably comprised in a relative weight amount of 0.1 to 0.5 (% w/w), more preferably in a relative weight amount of 0.3-0.4% (w/w).
15. The composition according to embodiment 13 or 14, wherein the composition consists of trehalose or sucrose, poloxamer 188, methionine, a buffer, and optionally NaCl, and optionally EDTA.
16. The composition according to any one of embodiments 1 to 6, comprising botulinum toxin, trehalose or sucrose, polysorbate 20, and methionine.
17. The composition according to embodiment 16, wherein the relative weight amounts (%, w/w) of trehalose or sucrose, polysorbate 20, and methionine are within the following ranges:

| | |
|---|---|
| trehalose or sucrose | 1to 10 |
| polysorbate 20 | 0.02 to 0.06 |
| methionine | 0.1 to 0.3 |

18. The composition according to any one of embodiments 1 to 6, comprising botulinum toxin, trehalose or sucrose, poloxamer 188, and N-Acetyl-cysteine.
19. The composition according to embodiment 18, wherein the relative weight amounts (%, w/w) of trehalose or sucrose, poloxamer 188, and N-Acetyl-cysteine are within the following ranges:

| | |
|---|---|
| trehalose or sucrose | 1 to 10 |
| poloxamer 188 | 0.5 to 5 |
| N-Acetyl-cysteine | 0.01 to 0.5, and optionally |
| EDTA | 0.01 to 0.1. |

20. The composition according to embodiment 19, wherein the relative weight amounts (%, w/w) of trehalose or sucrose, poloxamer 188, N-Acetyl-cysteine, and EDTA are:

| | |
|---|---|
| trehalose or sucrose | 7 to 9 |
| poloxamer 188 | 3.5 to 4.5 |
| N-Acetyl-cysteine | 0.1 to 0.3 and optionally |
| EDTA | 0.01 to 0.05; |

and are preferably

| | |
|---|---|
| trehalose or sucrose | 8 |
| poloxamer 188 | 4 |
| N-Acetyl-cysteine | 0.2 and optionally |
| EDTA | 0.03. |

21. The composition according to embodiment 19, wherein the relative weight amounts (%, w/w) of trehalose or sucrose, poloxamer 188, and N-Acetyl-cysteine are:

| | |
|---|---|
| trehalose or sucrose | 1 to 3 |
| poloxamer 188 | 3.5 to 4.5 |
| N-Acetyl-cysteine | 0.01 to 0.05; |

and are preferably:

| | |
|---|---|
| trehalose or sucrose | 2 |
| poloxamer 188 | 4 |
| N-Acetyl-cysteine | 0.03. |

22. The composition according to any one of embodiments 18 - 21, wherein the composition is a solid composition, wherein the composition is preferably lyophilised.
23. The composition according to embodiment 22, further comprising a buffer, wherein the buffer is preferably histidine, wherein the histidine is preferably comprised in a relative weight amount of 0.1 to 0.5 (% w/w), more preferably in a relative weight amount of 0.3-0.4% (w/w).
24. The composition according to embodiment 22 or 23, wherein the composition consists of trehalose or sucrose, poloxamer 188, N-Acetyl-cysteine, and a buffer.
25. The composition according to any one of embodiments 18 - 20, wherein the composition is a liquid composition, wherein the composition preferably comprises a buffer.
26. The composition according to embodiment 25, wherein the buffer is histidine, wherein the concentration of histidine is preferably 20 mM, wherein the pH is preferably in the range 5-7, and wherein the pH is most preferably 6.
27. The composition according to embodiment 25, wherein the composition consists of trehalose or sucrose, poloxamer 188, N-Acetyl-cysteine, a buffer, and optionally EDTA, wherein the buffer is preferably histidine, wherein the pH is preferably in the range 5-7, and wherein the pH is most preferably 6.
28. The composition according to any one of embodiments 1 to 6, comprising botulinum toxin, trehalose or sucrose, polysorbate 20, and N-Acetyl-cysteine.
29. The composition according to embodiment 28, wherein the relative weight amounts (%, w/w) of trehalose or sucrose, polysorbate 20, and N-Acetyl-cysteine are within the following ranges:

| | |
|---|---|
| trehalose or sucrose | 1to 10 |
| polysorbate 20 | 0.02 to 0.06 |
| N-Acetyl-cysteine | 0.01 to 0.5. |

30. A pharmaceutical composition comprising:
(i) a Clostridial toxin active ingredient;
(ii) a poloxamer and/or a polysorbate; and
(iii) an antioxidant,
wherein no sugar or polyalcohol is present in the composition.
31. The composition according to embodiment 30, comprising botulinum toxin.
32. The composition according to embodiment 30 or 31, comprising poloxamer 188 and/or polysorbate 20.
33. The composition according to any one of embodiments 30 to 32, comprising one or more of methionine and N-Acetyl-cysteine.
34. The composition according to any one of embodiments 30 to 33, comprising botulinum toxin, one of poloxamer 188 or polysorbate 20, and one of methionine or N-Acetyl-cysteine.
35. The composition according to any one of embodiments 30 to 34, comprising botulinum toxin, poloxamer 188, and methionine.
36. The composition according to embodiment 35, wherein the relative weight amounts (%, w/w) of poloxamer 188, and methionine are within the following ranges:

| | |
|---|---|
| poloxamer 188 | 0.5 to 5 |
| methionine | 0.1 to 0.3. |

37. The composition according to embodiment 36, wherein the relative weight amounts (%, w/w) of poloxamer 188, and methionine are:

| | |
|---|---|
| poloxamer 188 | 3.5 to 4.5 |
| methionine | 0.15 to 0.25; |

and are preferably

| | |
|---|---|
| poloxamer 188 | 4 |
| methionine | 0.2. |

38. The composition according to any one of embodiments 35 - 37, wherein the composition is a liquid composition, wherein the composition preferably comprises a buffer.
39. The composition according to embodiment 38, wherein the buffer is histidine, wherein the concentration of histidine is preferably 20 mM, wherein the pH is preferably in the range 5-7, and wherein the pH is most preferably 6.
40. The composition according to embodiment 38, wherein the composition consists of poloxamer 188, methionine, and a buffer, wherein the buffer is preferably histidine, wherein the pH is preferably in the range 5-7, and wherein the pH is most preferably 6.
41. The composition according to any one of embodiments 1 to 10, 12 to 25, 27 to 38, or 40 comprising histidine.
42. The composition according to any previous embodiment, comprising no animal-derived protein.
43. The composition according to any previous embodiment, comprising ethylene diamine tetraacetic acid sodium salt (EDTA) or an EDTA analog.
44. The composition according to embodiment 43, wherein the relative weight amount (%, w/w) of EDTA ranges from about 0.01 to 0.10.
45. The composition according to any one of embodiments 1 to 9, 16 to 21, 28, 29 and 41 to 44, wherein the composition is a solid formulation.
46. The composition according to any one of embodiments 19, 29, 43, or 44, wherein the composition is a solid formulation and the relative weight amount (%, w/w) of N-Acetyl-cysteine is 0.01 to 0.05.
47. The composition according to any one of embodiments 1 to 12, 16 to 21, and 25 to 44 wherein the composition is a liquid formulation and has a pH of from 5 to 7.
48. The composition according to any one of embodiments 18, 19, 28, 29, 43 and 44, wherein the composition is a liquid formulation and has a pH of from 5 to 7, wherein the relative weight amount of N-Acetyl-cysteine is 0.1 to 0.5.
49. A method of treating, reduction of symptoms, and/or prevention of diseases, disorders, and conditions, wherein the method comprises the step of administering the pharmaceutical composition of any preceding embodiment to a subject in need thereof.
50. The method of embodiment 49, wherein the diseases, disorders, and conditions are selected from neuromuscular diseases, pain, psychological disorders, urological disorders, inflammation, and skin disorders.
51. The method of embodiment 49, wherein the disorder is depression.
52. The method of embodiment 49, wherein the condition is cardiac arrhythmia.
53. A method of cosmetic treatment comprising the step of administering the pharmaceutical composition of any of embodiments 1 to 48 to a subject in need thereof. The present invention relates to the following items 1 to 44:
1. A pharmaceutical composition comprising:
(i) a botulinum toxin;
(ii) poloxamer 188; and
(iii) methionine or N-Acetyl-cysteine.
2. The composition according to item 1, further comprising trehalose or sucrose, and optionally NaCl, and optionally EDTA.
3. The composition according to item 2, wherein the relative weight amounts (%, w/w) of trehalose or sucrose, poloxamer 188, methionine, and optionally NaCl, and optionally EDTA are within the following ranges:

| | |
|---|---|
| trehalose or sucrose | 1 to 10 |
| poloxamer 188 | 0.5 to 5 |
| methionine | 0.1 to 0.3 and optionally |
| NaCl | 0.1 to 10 and optionally |
| EDTA | 0.01 to 0.1. |

4. The composition according to item 3, wherein the relative weight amounts (%, w/w) of trehalose, poloxamer 188, methionine, and optionally NaCl, and optionally EDTA are:

| a) | trehalose or sucrose | 7 to 9 |
|---|---|---|
| | poloxamer 188 | 3.5 to 4.5 |
| | methionine | 0.15 to 0.25; |
| and are preferably | trehalose or sucrose | 8 |
| | poloxamer 188 | 4 |
| | methionine | 0.2; or |

| b) | trehalose or sucrose | 7 to 9 |
|---|---|---|
| | poloxamer 188 | 0.5 to 0.7 |
| | methionine | 0.15 to 0.25; |
| and are preferably | trehalose or sucrose | 8 |
| | poloxamer 188 | 0.6 |
| | methionine | 0.2; or |

| c) | trehalose or sucrose | 1 to 3 |
|---|---|---|
| | poloxamer 188 | 3.5 to 4.5 |
| | methionine | 0.15 to 0.25 |
| | NaCl | 0.4 to 0.8; |
| and are preferably | trehalose or sucrose | 2 |
| | poloxamer 188 | 4 |
| | methionine | 0.2 |
| | NaCl | 0.6; or |

| d) | trehalose or sucrose | 7 to 9 |
|---|---|---|
| | poloxamer 188 | 3.5 to 4.5 |
| | methionine | 0.15 to 0.25 |
| | EDTA | 0.01 to 0.05; |
| and are preferably | trehalose or sucrose | 8 |
| | poloxamer 188 | 4 |
| | methionine | 0.2 |
| | EDTA | 0.03. |

5. The composition according to item 2, wherein the relative weight amounts (%, w/w) of trehalose or sucrose, poloxamer 188, N-Acetyl-cysteine and EDTA are within the following ranges:

| | |
|---|---|
| trehalose or sucrose | 1 to 10 |
| poloxamer 188 | 0.5 to 5 |
| N-Acetyl-cysteine | 0.01 to 0.5, and |
| EDTA | 0.01 to 0.1. |

6. The composition according to item 5, wherein the relative weight amounts (%, w/w) of trehalose, poloxamer 188, N-Acetyl-cysteine, and EDTA are:

| | |
|---|---|
| trehalose or sucrose | 7 to 9 |
| poloxamer 188 | 3.5 to 4.5 |
| N-Acetyl-cysteine | 0.1 to 0.3 and |
| EDTA | 0.01 to 0.05; |

and are preferably

| | |
|---|---|
| trehalose or sucrose | 8 |
| poloxamer 188 | 4 |
| N-Acetyl-cysteine | 0.2 and |
| EDTA | 0.03. |

7. The composition according to any previous item, comprising trehalose.
8. A pharmaceutical composition according to item 1, wherein the composition does not comprise a sugar or polyalcohol.
9. The composition according to item 8, wherein the relative weight amounts (%, w/w) of poloxamer 188, and methionine are within the following ranges:

| | |
|---|---|
| poloxamer 188 | 0.5 to 5 |
| methionine | 0.1 to 0.3. |

10. The composition according to item 9, wherein the relative weight amounts (%, w/w) of poloxamer 188, and methionine are:

| | |
|---|---|
| poloxamer 188 | 3.5 to 4.5 |
| methionine | 0.15 to 0.25; |

and are preferably

| | |
|---|---|
| poloxamer 188 | 4 |
| methionine | 0.2. |

11. The composition according to any one of items 1 - 10, wherein the composition is a liquid composition, wherein the composition preferably comprises a buffer.
12. The composition according to item 11, wherein the buffer is histidine, wherein the concentration of histidine is preferably 20 mM, wherein the pH is preferably in the range 5-7, and wherein the pH is most preferably in the range 5.5-6.
13. The composition according to any one of items 1 - 7, wherein the composition is a solid composition, wherein the comosition is preferably lyophilised.
14. The composition according to item 13, further comprising a buffer, wherein the buffer is preferably histidine, wheren the histidine is preferably comprised in a relative weight amount of 0.1 to 0.5 (% w/w), more preferably in a relative weight amount of 0.3-0.4% (w/w).
15. The composition according to any one of items 1 to 4, wherein the composition is a liquid composition consisting of one or more botulinum toxins, trehalose or sucrose, poloxamer 188, methionine, a buffer, and optionally NaCl, and optionally EDTA.
16. The composition according to any one of items 1 to 4, wherein the composition is a solid composition consisting of one or more botulinum toxins, trehalose, poloxamer 188, methionine, a buffer, and optionally NaCl, and optionally EDTA.
17. The composition according to any one of items 1, 2, 5 and 6, wherein the composition is a liquid composition consisting of one or more botulinum toxins, trehalose or sucrose, poloxamer 188, N-Acetyl-cysteine, a buffer, and EDTA.
18. The composition according to any one of items 1, and 8 to 10, wherein the composition is a liquid composition and consists of one or more botulinum toxins, poloxamer 188, methionine, and a buffer.
19. The composition according to any previous item, comprising no animal-derived protein.
20. The composition according to any one of items 1 to 17 and 19, comprising ethylene diamine tetraacetic acid sodium salt (EDTA) or an EDTA analog.
21. The composition according to item 20, wherein the relative weight amount (%, w/w) of EDTA ranges from about 0.01 to 0.10.
22. A liquid pharmaceutical composition, comprising:
(i) a Clostridial toxin;
(ii) a poloxamer;
(iii) a chelating agent; and
(iv) a sacrificial antioxidant.
23. The liquid pharmaceutical composition according to item 22, wherein the chelating agent is EDTA, EGTA or DTPA, and the sacrificial antioxidant is ascorbic acid.
24. A liquid pharmaceutical composition, comprising:
(i) a Clostridial toxin;
(ii) a poloxamer;
(iii) a chelating agent; and
(iv) a chain terminator.
25. The liquid composition according to item 24, wherein the chelating agent is EDTA and the chain terminator is N-acetyl-cysteine.
26. The liquid composition according to item 24, wherein the chelating agent is EDTA and the chain terminator is Butylated hydroxytoluene.
27. A liquid pharmaceutical composition, comprising:
(i) a Clostridial toxin;
(ii) a poloxamer; and
(iii) methionine.
28. A liquid pharmaceutical composition, comprising:
(i) a Clostridial toxin;
(ii) a tonicity agent selected from trehalose, sucrose, sodium chloride, mannitol, sorbitol, glucose, and combinations thereof;
(iii) a poloxamer; and
(iv) methionine.
29. A liquid pharmaceutical composition, comprising:
(i) a Clostridial toxin;
(ii) a tonicity agent selected from trehalose, sucrose, sodium chloride, mannitol, sorbitol, glucose, and combinations thereof;
(iii) a surfactant selected from a poloxamer, a polysorbate and combinations thereof;
(iv) a chelating agent; and
(v) a chain terminator.
30. A lyophilized pharmaceutical composition, comprising:
(i) a Clostridial toxin;
(ii) trehalose, sucrose, mannitol, sorbitol, glucose, or combinations thereof;
(iii) a surfactant selected from a poloxamer, a polysorbate and combinations thereof;
(iv) a chelating agent; and
(v) a sacrificial antioxidant.
31. A lyophilized pharmaceutical composition, comprising:
(i) a Clostridial toxin;
(ii) trehalose, sucrose, mannitol, sorbitol, glucose, or combinations thereof;
(iii) a poloxamer;
(iv) a chelating agent; and
(v) a sacrificial antioxidant.
32. The composition according to any one of items 30 to 31, wherein the chelating agent is EDTA, EGTA or DTPA, and the sacrificial antioxidant is ascorbic acid.
33. A lyophilized pharmaceutical composition, comprising:
(i) a Clostridial toxin;
(ii) trehalose, sucrose, mannitol, sorbitol, glucose, or combinations thereof;
(iii) a poloxamer;
(iv) a chelating agent; and
(v) a chain terminator.
34. A lyophilized pharmaceutical composition, comprising:
(i) a Clostridial toxin;
(ii) trehalose, sucrose, mannitol, sorbitol, glucose, or combinations thereof;
(iii) a surfactant selected from a poloxamer, a polysorbate and combinations thereof;
(iv) a chelating agent; and
(v) a chain terminator.
35. The lyophilized pharmaceutical composition according to any one of items 33-34, wherein the chelating agent is EDTA and the chain terminator is N-Acetyl-cysteine.
36. A lyophilized pharmaceutical composition, comprising:
(i) a Clostridial toxin;
(ii) trehalose, sucrose, mannitol, sorbitol, glucose, or combinations thereof;
(iii) a poloxamer; and
(iv) a chain terminator.
37. A lyophilized pharmaceutical composition, comprising:
(i) a Clostridial toxin;
(ii) trehalose, sucrose, mannitol, sorbitol, glucose, or combinations thereof;
(iii) a surfactant selected from a poloxamer, a polysorbate and combinations thereof; and
(iv) a chain terminator.
38. The lyophilized pharmaceutical composition according to any one of items 36-37, wherein the chain terminator is N-Acetyl-cysteine.
39. A lyophilized pharmaceutical composition, comprising:
(i) a Clostridial toxin;
(ii) trehalose, sucrose, mannitol, sorbitol, glucose, or combinations thereof;
(iii) a poloxamer; and
(iv) methionine.
40. A method of treating, reducing the symptoms, and/or prevention of diseases, disorders, and conditions, wherein the method comprises the step of administering the pharmaceutical composition of any preceding item to a subject in need thereof.
41. The method of item 40, wherein the diseases, disorders, and conditions are selected from neuromuscular diseases, pain, psychological disorders, urological disorders, inflammation, and skin disorders.
42. The method of item 41, wherein the disorder is depression.
43. The method of item 41, wherein the condition is cardiac arrhythmia.
44. A method of cosmetic treatment comprising the step of administering the pharmaceutical composition of any of items 1 to 39 to a subject in need thereof.

## Claims

1. A pharmaceutical composition comprising:
(i) a botulinum toxin;
(ii) poloxamer 188; and
(iii) methionine or N-Acetyl-cysteine.
